# FASCICULE DE BREVET EUROPEEN

(11) **EP 4 301 238 B1**
(45) Date de publication et mention de la délivrance du brevet: **30.09.2026**
(21) Numéro de dépôt: 21799079.5
(22) Date de dépôt: 08.09.2021
(51) Int. Cl.: A61B 10/00, A61B 5/20, G01F 1/708, G01F 1/56

(54) **CAPTEUR DE VOLUME ET DE DÉBIT, INJECTEUR ASSOCIÉ**
VOLUMEN- UND DURCHFLUSSSENSOR UND ZUGEHÖRIGES INJEKTIONSGERÄT
VOLUME AND FLOW SENSOR AND ASSOCIATED INJECTOR

(30) Priorité: 03.03.2021 WO PCT/EP2021/055302
(43) Date de publication de la demande: 10.01.2024
(73) Titulaire: Withings, 92130 Issy-Les-Moulineaux (FR)
(72) Inventeur: KIRAT, Marine, 92130 ISSY LES MOULINEAUX (FR); TINÉ, Jorys, 92130 ISSY LES MOULINEAUX (FR); JAMES, Leo, 92130 ISSY LES MOULINEAUX (FR); DUMESNIL DE MARICOURT, Etienne, 92130 ISSY LES MOULINEAUX (FR); RAFIK, Walid, 92130 ISSY LES MOULINEAUX (FR)
(74) Mandataire: Withings IP
(86) Numéro de dépôt international: PCT/FR2021/051542
(87) Numéro de publication internationale: WO 2022/184984

(56) Documents cités:
- US-A- 3 621 715
- US-A- 4 559 831
- US-A1- 2005 210 962
- US-A1- 2009 126 505
- US-A1- 2021 023 557

## Description

### Domaine technique

La présente divulgation relève du domaine des capteurs de débit et des capteurs de volume. Le domaine est aussi celui des injecteurs intégrant un tel capteur. Le domaine est aussi celui des circuits fluidiques intégrant une pompe pilotée par un tel capteur. Les quantités de fluide injecté sont typiquement de l'ordre de la dizaine de microlitres mais l'enseignement peut s'appliquer à tout volume.

Une application particulière de l'injecteur de la présente divulgation concerne les fluides dits mixtes, c'est-à-dire les fluides comprenant du liquide et du gaz (par exemple des alternances de fronts liquide-gaz) ou deux liquides différents. Par bulle, on entend un faible volume de gaz inclus dans un front de liquide (qui ne sépare la phase liquide en deux phases). Typiquement, une miction d'urine peut être un fluide mixte, notamment lorsque la collecte n'est pas contrôlée. L'injecteur ne nécessite donc pas de débulleur dédié pour supprimer l'alternance liquide-gaz. L'injection de faible quantité de liquide à partir d'une récole de fluide mixte génère des difficultés de précision et de répétabilité. L'injecteur de la présente description peut être utilisé dans un dispositif d'analyse d'urine destinés à être positionnés à l'intérieur de toilettes, partiellement ou complètement.

### Technique antérieure

Il existe différents types de capteurs pour mesurer un volume et/ou un débit. Outre les débitmètres classiques à éléments tournants, il existe des capteurs utilisant des électrodes.

Le document US2007251330 décrit un tube avec un corps qui peut se déplacer dans le tube. Le tube comprend un émetteur optique et une paire de récepteurs optiques qui peuvent repérer la présence du corps à son passage. Le temps de passage du corps devant des capteurs adjacents peut être déterminés, ce qui permet de connaitre le débit massique ou volumétrique.

Le document US2007119261 décrit l'utilisation d'électrodes pour identifier la présence de fluide dans un conduit. En utilisant plusieurs paires d'électrodes à la suite et l'écart de temps entre les variations de signaux pour les paires d'électrodes, il est possible de déterminer le débit dans le conduit. Il est indiqué que cette méthode convient pour les interfaces gaz-liquide ou liquide-liquide (avec des permittivités différentes).

Le document US2005210962 décrit un système d'analyse microfluidique, pour notamment de l'urine. Le système comprend plusieurs électrodes en série dans un conduit. Deux électrodes peuvent être utilisés, avec un chronomètre, pour calculer un débit. Trois électrodes peuvent être utilisés pour notamment améliorer la précision du débit et pour détecter des bolus de liquide.

Les documents US3621715, US4559831 et US2009/126505 décrivent également différentes architectures de débitmètres. Le document US2005/0210962 décrit un système d'analyse microfluidique avec un micro-canal et au moins une électrode de position.

Néanmoins, ces dispositifs présentent des limitations qui ne le rendent pas utilisables, notamment dans le cas d'un analyseur d'urine monté dans des toilettes. De plus, la mesure du débit n'est pas toujours suffisante dans le cas d'un fluide mixte. De la même façon, la mesure du volume ne permet pas toujours de s'affranchir des problématiques de l'alternance liquide-air. La question d'encombrement est aussi importante, notamment pour des circuits fluidiques compacts (dans le cas d'injection de microlitres à quelques dizaines de microlitres).

### Résumé

La présente description vise à proposer une ou plusieurs solutions résolvant au moins certains des inconvénients précités.

En particulier, le déposant a développé un dispositif d'analyse d'urine comprenant une station et une cartouche (aussi appelé support rotatif), décrit dans les documents PCT/EP2021055302 et PCT/EP2021/055377. La présente description propose un capteur, un injecteur et un circuit fluidique qui peuvent être montés sur cette station.

L'invention est définie dans la revendication 1.

Dans un aspect, l'invention propose un capteur volumétrique comprenant un conduit à l'intérieur duquel un fluide mixte peut circuler. Par fluide mixte, il est signifié un fluide non homogène, soit par alternance de liquides non-miscibles, soit par alternance de liquide et de gaz. Le conduit reçoit une première sonde qui peut identifier un changement de phase du fluide mixte dans le conduit, lorsque le fluide passe au niveau de la première sonde. Similairement, le conduit reçoit une deuxième sonde qui peut identifier un changement de phase du mixte dans le conduit, lorsque le fluide passe au niveau de la deuxième sonde. La deuxième sonde est espacée de la première sonde le long du conduit. Typiquement, si on définit un amont/aval relatif au sens d'écoulement du fluide ou un proximal/distale, relatif à sens de déplacement du fluide, la première sonde se situe en amont et la deuxième sonde se situe en aval. La première sonde est utilisée pour définir un volume de référence Vref du conduit. Le capteur comprend en outre une unité de contrôle électronique ECU comprenant des instructions pour déterminer que le volume de référence est empli de liquide et pour déterminer une valeur grandeur physique de déplacement liée au déplacement du liquide entre la première sonde et la deuxième sonde. La grandeur physique de déplacement est typiquement un temps ou un nombre de coups de pompe, la pompe mettant le fluide en déplacement au sein du conduit. Pour déterminer que le volume de référence est empli de liquide, l'ECU peut identifier les changements de phase au niveau de la première sonde (au moins).

On suppose ici que le capteur est vide à son activation. De la sorte, on est assuré que l'ECU peut établir une correspondance de changement de phase entre les changements de phase détectés par la première sonde et la deuxième sonde.

La grandeur physique de déplacement est un temps ou un nombre de coups de pompe, la pompe mettant le fluide en mouvement dans le conduit.

En outre, la première position P1 et la deuxième position P2 peuvent définir un volume de contrôle du conduit, l'ECU comprenant des instructions pour déterminer un débit en utilisant la valeur déterminée de la grandeur physique de déplacement déterminée (Nb, T) et la valeur du volume de contrôle Vc

L'ECU comprend des instructions pour effectuer les étapes suivantes :
E1 détection d'un changement de phase vers une phase liquide, par la première sonde,
E2 détection d'un passage d'un changement de phase vers une phase liquide par la deuxième sonde.
E3 détermination, à l'aide d'un compteur, de la valeur de la grandeur physique de déplacement (N) (durée ou coups de pompe par exemple), qui correspond à la grandeur physique écoulée entre la détection du changement de phase par la première sonde et la détection du changement de phase par la deuxième sonde.

Si la position P2 est en amont de la position P1, l'étape E2 est faite avant l'étape E1.

L'étape de détermination de la valeur de la grandeur physique de déplacement peut comprendre E3 :
- en réponse à l'étape de détection E1 par la première sonde, E31 démarrage d'un compteur, ledit compteur étant associé au changement de phase détecté,
- en réponse à l'étape de détection E2 par la deuxième sonde, E32 arrêt du chronomètre associé au changement de phase détecté.

L'ECU peut stocker une valeur du volume de contrôle Vc et des instructions pour effectuer les étapes suivantes :
- E4 calcul d'un débit en utilisant la valeur déterminée (N) de la grandeur physique de déplacement déterminée (Nb, T) et la valeur du volume de contrôle Vc.

Les instructions peuvent comprendre en outre E5 la génération d'une consigne d'injection pour une pompe en utilisant le débit déterminé et une consigne prédéterminée, pour injecter une quantité souhaitée de liquide. L'étape de génération de consigne d'injection E5 peut comprendre une conversion entre le débit et la consigne d'injection.

Le volume de contrôle Vc est égal à ce dernier.

En particulier, le capteur se décline en deux modes de réalisation principaux.

Dans un premier mode de réalisation, la détermination que le volume de référence est empli de liquide et la détermination de la valeur de la grandeur physique de déplacement sont effectuées en même temps.

Dans ce mode de réalisation, aussi dit « absolu », les sondes peuvent mesurent le changement de phase localement dans le conduit (à une abscisse donnée). Dans ce mode de réalisation, la première sonde et la deuxième sonde définissent entre elles le volume de référence Vref. En d'autres termes, la portion de conduit qui se trouvent entre les deux sondes est le volume de référence. Dans ce mode de réalisation toujours, la détermination que le volume de référence est empli de liquide se fait en identifiant les changements de phase au niveau de la première sonde et de la deuxième sonde.

La détermination que le volume de référence Vref est empli de liquide se fait alors en identifiant les changements de phase par la première sonde et de la deuxième sonde.

L'ECU peut alors comprendre des instructions pour effectuer les étapes suivantes :
- E2a détermination que la première sonde n'a pas détecté d'autre changement de phase entre, temporellement, les deux détections E1, E2 de changement de phase par la première sonde et la deuxième sonde,
- E2b en réponse à la détermination de l'étape E2a, détermination que le volume de référence Vref est empli de liquide.

Dans un deuxième mode de réalisation, qui n'est pas couvert par les revendications, la détermination que le volume de référence est empli de liquide est effectuée avant ou après la détermination de la valeur de la grandeur physique de déplacement, mais pas en même temps (c'est-à-dire pas avec les mêmes volumes physiques du capteur).

Dans ce mode de réalisation, aussi dit « relatif », qui n'est pas couvert par les revendications, les sondes comprennent une paire d'électrodes et mesurent le changement de phase entre deux abscisses données. Dans ce mode de réalisation, la première sonde comprend une première paire d'électrodes connectées électriquement et disposés espacées le long du conduit. Elles définissent le volume de référence. Entre d'autres termes, la portion de conduit qui se trouve entre les deux électrodes est le volume de référence. Dans ce mode de réalisation, la deuxième sonde comprend une deuxième paire d'électrodes connectée électriquement et disposées espacées le long du conduit. La deuxième paire est distincte de la première paire (i.e. au moins une électrode diffère). Dans ce mode de réalisation, la détermination que le volume de référence est empli de liquide se fait en identifiant les changements de phase au niveau de la première sonde. Plus spécifiquement, si la conductivité de la première paire augmente, il est identifié que le volume de référence est empli de liquide.

Ainsi, la première sonde comprend une première paire d'électrodes, comprenant une première électrode amont et une première électrode aval disposées espacées le long du conduit et connectées électriquement, définissant entre elles le volume de référence Vref, la première électrode aval définissant la première position P1,

La détermination que le volume de référence Vref est empli de liquide se fait en identifiant un changement de phase vers une phase liquide par la première sonde.

La deuxième sonde peut comprendre une deuxième paire d'électrodes une deuxième électrode amont et une deuxième électrode aval, disposées espacées le long du conduit et connectées électriquement, distincte de la première paire d'électrodes (au moins une électrode qui diffère), la deuxième électrode aval définissant la deuxième position P2,

La deuxième sonde peut être est une sonde optique, thermique ou capacitive.

La première paire d'électrodes et la deuxième paire d'électrodes peuvent partager une électrode : la première électrode amont et la deuxième électrode amont sont communes, ou la première électrode aval et la deuxième électrode amont sont communes. La deuxième électrode aval est par exemple en aval de la première électrode aval. Le volume de contrôle Vc peut être adjacent au volume de référence Vref. Le volume de contrôle Vc est défini entre la première électrode aval de la première paire et la deuxième électrode aval de la deuxième paire.

Dans un autre aspect, l'invention se rapporte à un injecteur comportant le capteur tel que décrit précédemment.

L'injecteur peut comprendre un capteur de volume et de débit tel que décrit précédemment et une buse d'injection avec une extrémité distale d'injection, la buse d'injection étant connectée à une extrémité du conduit du capteur. La valeur du volume du conduit entre la deuxième position et l'extrémité distale d'injection, appelé volume mort Vm, peut être inférieure à la valeur du volume de référence Vref ou moins de deux fois la valeur du volume de référence.

L'ECU peut stocker la valeur du volume mort Vm et les instructions comprennent en outre l'étape suivante : la consigne d'injection utilise le débit déterminé, une consigne prédéterminée et le volume mort Vm.

L'ECU peut générer une consigne de pilotage d'un volume égal à au volume de la consigne prédéterminée plus le volume mort. Alternativement, l'ECU génère une consigne de précharge pour une pompe, d'un volume égal au volume mort Vm ou au (1+A%)Vm, A étant compris entre 0 et 50.

Dans un autre aspect, l'invention se rapport à un système d'injection fluidique, qui comprend une pompe et un capteur tel que décrit précédemment ou un injecteur tel que décrit précédemment, la pompe étant pilotée par l'ECU pour recevoir des consignes de précharge ou d'injection.

Dans un aspect, la description se rapporte aussi à une station pour dispositif d'analyse d'urine, la station comprenant :
- un boitier, destiné à être positionné à l'intérieur de toilettes,
- un logement annulaire, autour d'un axe de rotation, dans le boitier, le logement annulaire étant configuré pour recevoir au moins partiellement une cartouche montée à rotation autour de l'axe de rotation dans la station et comprenant une pluralité de supports de test de test,
- un injecteur tel que décrit précédemment, positionné dans le boitier, et configuré pour injecter un volume contrôlé d'urine sur au moins un support de test, l'injecteur étant monté mobile en translation par rapport au boitier.

Le boitier peut comprendre en outre une pompe telle que décrite précédemment.

Le boitier peut en outre comprendre un analyseur (par exemple un analyseur optique), positionné au moins partiellement radialement à l'extérieur du logement annulaire et configuré pour analyser optiquement au moins un support de test.

Le boitier peut comprendre un actionneur d'entrainement, apte à entrainer en rotation une cartouche telle que décrite précédemment, afin de positionner sélectivement les supports de test devant l'injecteur et l'analyseur.

La présente description se rapport aussi un dispositif d'analyse d'urine comprenant une station (telle que décrite ci-dessus) et une cartouche. La cartouche est alors configurée pour être au moins partiellement reçue dans le logement annulaire du boitier. Chaque support de test est solidaire de la cartouche et est configuré pour pouvoir être sélectivement positionné devant l'injecteur et l'analyseur. En particulier, le support de test est une bandelette de test. La présente description couvre aussi un kit comprenant cette station et une cartouche ou une pluralité de cartouches (cartouches avec différents types de supports de test).

### Brève description des dessins

D'autres caractéristiques, détails et avantages apparaîtront à la lecture de la description détaillée ci-après, et à l'analyse des dessins annexés, sur lesquels :
[Fig. 1] Cette figure représente schématiquement une vue partielle d'un injecteur comprenant un capteur selon un premier mode de réalisation.
[Fig. 2] Cette figure représente schématiquement une vue partielle d'un injecteur comprenant un capteur selon un deuxième mode de réalisation, qui n'est pas couvert par les revendications.
[Fig. 3] Cette figure représente schématiquement un circuit fluidique comprenant un injecteur selon le premier mode de réalisation et une pompe.
[Fig. 4] Cette figure représente une méthode conforme à un mode de réalisation.
[Fig. 5] Cette figure représente l'état du capteur de la figure 1 à différentes étapes.
[Fig. 6] Cette figure représente un mode de réalisation particulier d'une méthode de calcul de débit.
[Fig. 7] Cette figure représente l'état du capteur de la figure 2 à différentes étapes.
[Fig. 8] Cette figure représente un mode de réalisation particulier d'une méthode de calcul de débit.
[Fig. 9] Cette figure représente schématiquement une vue partielle d'un injecteur comprenant un capteur selon une variante du deuxième mode de réalisation de l'invention, non couvert par les revendications.
[Fig. 10] Cette figure représente schématiquement une vue partielle d'un injecteur comprenant un capteur selon une autre variante du deuxième mode de réalisation de l'invention, non couvert par les revendications.
[Fig. 11] Cette figure représente schématiquement une vue partielle d'un injecteur comprenant un capteur selon une autre variante du deuxième mode de réalisation de l'invention, non couvert par les revendications.
[Fig. 12] Cette figure représente l'état du capteur de la figure 1 à différentes étapes de la consigne d'activation (pré-charge).
[Fig. 13] Cette figure représente schématiquement une vue en coupe de toilettes équipées d'un dispositif d'analyse d'urine au sens de l'invention.
[Fig. 14] Cette figure représente une vue éclatée d'un mode de réalisation d'un dispositif d'analyse d'urine.
[Fig. 15] Cette figure représente une vue de côté d'une station ou d'un dispositif d'analyse d'urine selon un mode de réalisation.
[Fig. 16] Cette figure représente une cartouche coopérant avec la station pour former un dispositif d'analyse d'urine, avec une vue partielle sans la portion cylindrique pour mieux voir le séparateur.
[Fig. 17] Cette figure présente une vue en coupe de la station selon le plan orthogonal à l'axe de rotation.
[Fig. 18] Cette figure présente une vue schématique de certains composants du dispositif d'analyse d'urine et de son environnement.
[Fig. 19] Cette figure présente les étapes d'un procédé d'utilisation du dispositif d'analyse d'urine.

### Description des modes de réalisation

La présente description vise à présenter un capteur de volume et débit utilisable pour des fluides mixtes (liquide/gaz, liquide/liquide de natures différentes) en faible quantité (à partir de quelques microlitres). Le capteur peut être couplé à une buse d'injection pour former un injecteur. La proximité de la buse d'injection avec le capteur permet de profiter pleinement des avantages de ce dernier. En outre, comme le capteur permet de connaître un débit d'un fluide traversant le capteur, il permet de fait de connaitre le débit d'une pompe connectée audit capteur mettant en déplacement le fluide dans le capteur. Par conséquent, le capteur, lorsque couplé à une pompe, permet à une unité de contrôle électronique de piloter cette dernière pour obtenir une injection précise et répétable (rétroaction).

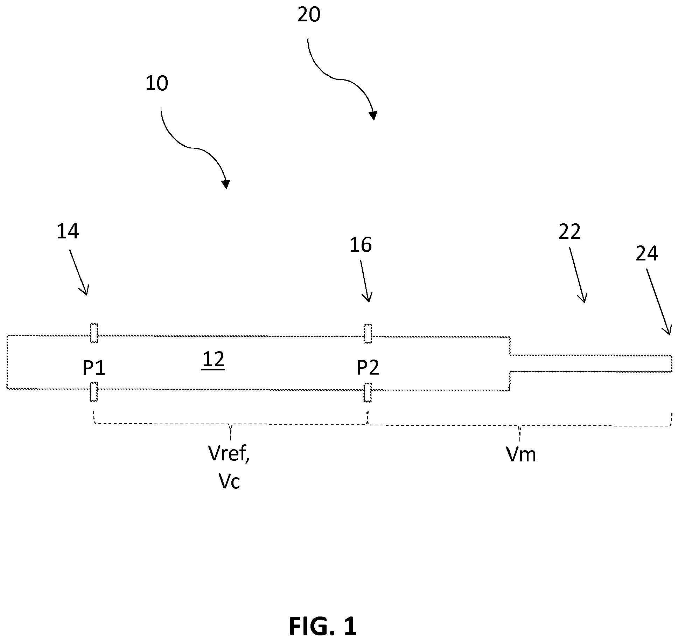

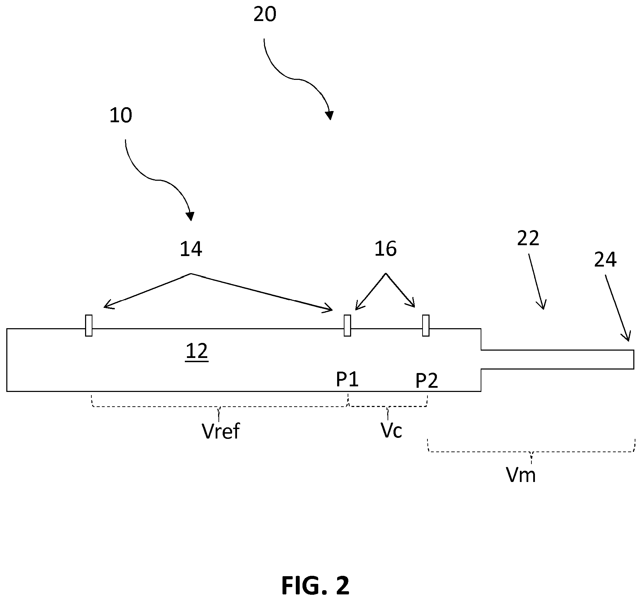

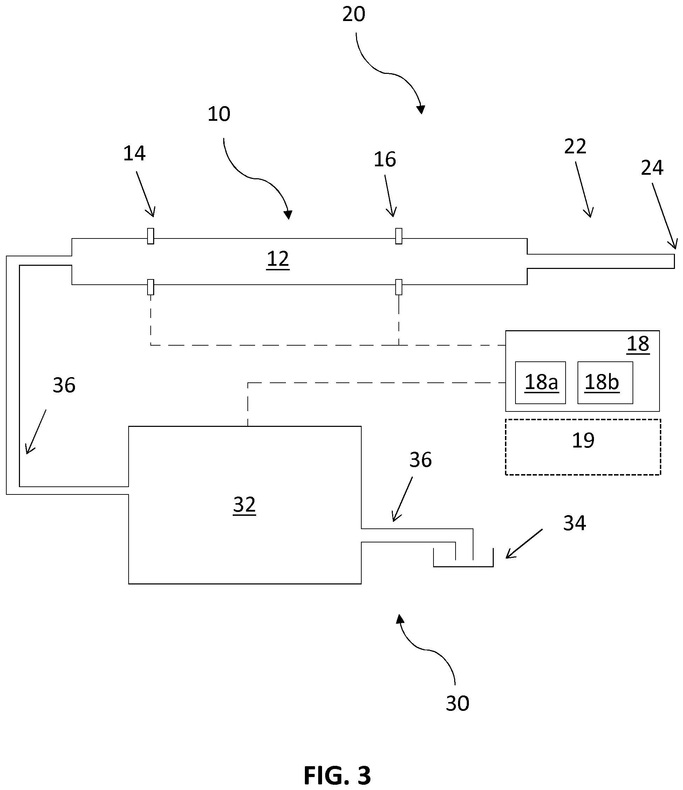

En particulier, les figures 1 et 2 illustrent deux modes de réalisation d'un capteur. Plus spécifiquement, les figures 1 et 2 illustrent une vue partielle de capteurs, qui sont montés sur un injecteur. Ces deux capteurs ou injecteurs peuvent être intégrés à un circuit fluidique comprenant une pompe, comme illustré en figure 3.

Le capteur 10 comprend un conduit 12 qui sert à guider et conduire le fluide mixte. Le capteur 10 a un sens de fonctionnement, de sorte que l'on peut définir un amont et un aval dans le conduit 12. Sur les figures, l'amont est à gauche et l'aval est à droite. Le capteur 10 comprend une première sonde 14 disposée à une première position P1 et une deuxième sonde 16 positionnée à une deuxième position P2. La première sonde 14 et la deuxième 16 permettent d'identifier un changement de phase du fluide mixte (notamment phase gazeuse vers phase liquide, phase liquide vers phase gazeuse, phase liquide vers phase liquide avec des propriétés différentes). Par exemple, elles peuvent identifier un changement de phase air/gaz ou liquide/liquide si les fluides ont des propriétés optiques, électriques ou thermiques différentes. La deuxième position P2 est espacées, le long du conduit 12, de la première position P1. En particulier, la première position P1 est en amont et la deuxième position est en aval. Par « disposée à une première (respectivement deuxième) position », il est entendu l'emplacement dans le conduit où la première (respectivement deuxième) sonde détecte effectivement le changement de phase.

La première sonde 14 et la deuxième sonde 16 (« les sondes ») sont connectées à une unité de contrôle électronique ECU 18 (visible en figure 3, qui représente le capteur de la figure 1, mais qui pourrait intégrer le capteur de la figure 2 ou tout autre variante de la présente description). L'ECU 18 est configurée pour instruire les sondes de prendre une mesure et pour recevoir et traiter les données reçues par les sondes. A cet égard, l'ECU comprend un processeur 18a et une mémoire 18b. La mémoire 18b peut stocker des instructions (lignes de code par exemple) que le processeur exécute pour mettre en oeuvre des étapes de procédé.

Pour alimenter en énergie les sondes 14, 16 et l'ECU 18, une source d'énergie 19 est prévue (par exemple une batterie rechargeable ou une alimentation secteur).

Sur les figures 1 et 2, le capteur 10 est intégré à un injecteur 20. L'injecteur 20 comprend une buse d'injection 22, avec une extrémité distale d'injection 24. La buse d'injection 22 est connectée au conduit 12, en particulier à une extrémité avale. Le long de l'injecteur 20, selon le mode de réalisation illustré en figures 1 et 2, il y a donc dans cet ordre : la première sonde 14, la seconde sonde 16, la buse d'injection 22. La buse d'injection 22 peut comprendre une aiguille.

Le capteur 10, qu'il soit intégré ou non à un injecteur 20, peut faire partie d'un circuit fluidique 30, illustré en figure 3. Le circuit hydraulique comprend une pompe 32 apte à prélever du fluide mixte depuis un réservoir 34 et à acheminer le fluide mixte vers le capteur 10 ou l'injecteur 20. Des conduits 36 assurent les connexions fluidiques entre le réservoir 34, la pompe 32 et le capteur 10/injecteur 20. La pompe 32 peut être commandée par l'ECU 18. La pompe 32 est typiquement alimentée en énergie par la source d'énergie 19. Le réservoir 34 peut comprendre un tuyau apte à stocker suffisamment de fluide mixte et/un collecteur (non représentés). Dans un mode de réalisation, le circuit fluidique fait entre 500 et 1000 microlitres. La compressibilité de l'air dans le circuit fluidique 30, lorsqu'il est empli de fluide mixte, est une des raisons liées à la difficulté d'injection d'un volume contrôlé.

La pompe 32 peut être une pompe piézoélectrique, dont le pilotage se fait généralement en coups de pompe, c'est à dire à chaque aller-retour d'un élément piézoélectrique mise en mouvement par l'application d'une tension à ses bornes. Modulo une correspondance nombre des coups de pompe et durée d'activation (durée d'activation = nombre de coups de pompe / fréquence de coup de pompe), le pilotage peut aussi se faire en durée. A chaque coup de pompe, la pompe pousse le liquide d'un volume donné. Le volume donné dépend de la pompe et de son régime de pompage. Ce volume donné n'est généralement pas connu. La connaissance de volume donné (via la connaissance du débit de la pompe) est un des objets de la présente description, grâce au capteur 10. On suppose que, pour la pompe 32, sur un régime de pompage donné, on suppose qu'il y a une relation linéaire entre le volume déplacé et le nombre de coup de pompe et/ou le temps d'activation.

Le conduit 12 peut avoir toute forme et/ou toute section. Une section circulaire ou ovale convient. Les dimensions peuvent être de quelques millimètres ou dixièmes de millimètre.

L'ECU 18 peut être réalisé à l'aide de plusieurs unités de contrôle électronique ECUs (comprenant chacun un processeur et une mémoire) communiquant entre elles. En particulier, le capteur 10 et/ou la pompe 32 peuvent chacun comprendre une unité de contrôle électronique intégrée, connectée à une unité de contrôle électronique principale du dispositif d'analyse. L'ECU 18 recouvre ici tout processeur et/ou mémoire utilisée pour le capteur 10 et la pompe 32.

La première position P1 est utilisée pour définir un volume de référence Vref du conduit 12 (avec une section du conduit 12). Le volume de référence Vref représente une portion du volume interne du conduit 12. Ce volume de référence Vref, qui sera décrit plus précisément pour les deux modes de réalisation principaux des figures 1 et 2, sert à assurer qu'il y a une quantité de liquide (ou d'un même liquide) suffisante, pour une utilisation donnée (par exemple pour une injection ultérieure par la buse d'injection 22). Par exemple, pour une injection désirée de 5 µL, on peut prévoir un volume de référence Vref compris entre 10 µL et 30 µL (par exemple 20µL) de sorte que l'on s'assure que l'injection se fasse uniquement avec du liquide et pas une alternance de liquide et de gaz.

Le capteur 10, en identifiant un changement de phase du fluide mixte, d'une phase de gaz vers une phase liquide ou d'une phase liquide vers une autre phase liquide (avec des propriétés différentes), par la première sonde 14, détermine que le volume de référence Vref est empli de liquide ou d'un même liquide. Davantage de détails seront donnés pour les modes de réalisation des figures 1 et 2.

De plus, le capteur 10 est configuré pour déterminer une valeur N d'une grandeur physique de déplacement T, Nb liée au déplacement d'un front de liquide entre la première position P1 et la deuxième position P2 (entre les deux sondes donc). La grandeur physique de déplacement peut être une durée T (en seconde par exemple) ou un nombre de coup de pompe Nb dans le cas du circuit fluidique 30 de la figure 3.

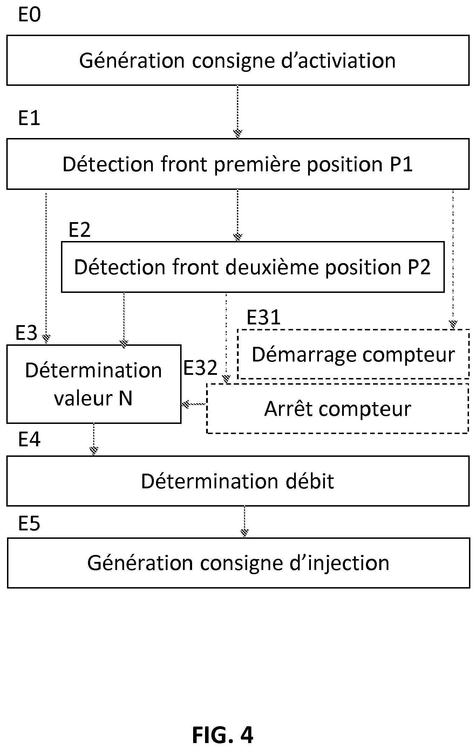

En particulier, comme illustré en figure 4, l'ECU peut être configurée pour mettre en oeuvre les étapes suivantes :
- (E1) détection d'un changement de phase (vers liquide, donc de gaz vers liquide ou de liquide vers liquide avec des propriétés différentes) par la première sonde 14,
- (E2) détection d'un changement de phase (vers liquide, donc de gaz vers liquide ou de liquide vers liquide avec des propriétés différentes) par la deuxième sonde 16,
- (E3) détermination, à l'aide d'un compteur et des détections des étapes E1 et E2, de la valeur N de la grandeur physique de déplacement T, Nb (une durée ou un nombre de coups de pompe 32 typiquement).

Une étape préliminaire E0 peut être générée par le capteur, de consigne d'activation de la pompe 32, pour mettre le fluide mixte en déplacement dans le capteur 10.

Il est supposé que le capteur 10 est vide avant toute utilisation. De la sorte, une correspondance de changements de phase entre les détections par la première sonde et la deuxième sonde peut être établie par l'ECU : tout changement de phase détecté par la deuxième sonde 16 a été détecté par la première sonde 14. Le changement de phase détecté à l'étape E2 correspond au même changement de phase que celui détecté à l'étape E1.

L'étape E3 peut comprendre une sous-étape E31 de démarrage du compteur à la détection d'un changement de phase par la première sonde (étape E1) et une sous-étape E31 d'arrêt du compteur à la détection d'un changement de phase par la deuxième sonde (étape E2). Le compteur est associé au changement de phase, ce qui permet de lancer plusieurs compteurs en parallèle si la première sonde détecte un premier changement de phase puis un deuxième changement de phase avant que la deuxième sonde n'ait détecté le premier changement de phase.

La première position P1 et la deuxième position P2 définissent dans le conduit 12 un volume de contrôle Vc, qui est connu de l'ECU (section et distance entre P1 et P2 connues). Par conséquent, grâce à valeur déterminée N de la grandeur physique de déplacement Nb, T et grâce à la valeur du volume de contrôle Vc, le capteur 10 peut calculer un débit (étape E4). En fonction de l'unité de la valeur déterminée N de la grandeur physique de déplacement Nb, T, le débit peut être un volume par unité de temps ou un volume par coup de pompe.

Par ailleurs, une fois l'étape E2 effectuée, l'ECU peut envoyer une consigne d'arrêt de la pompe 32 (soit d'arrêt définitif, soit de temporisation pour que l'ECU puisse terminer les calculs) ou bien envoyer, une fois l'étape E4 mise en oeuvre, une consigne de pré-charge qui sera décrite par la suite.

En fonction des modes de réalisation qui vont être décrits par la suite, le volume de contrôle Vc peut être le même que le volume de référence Vref, ou, si différent, peut être choisi pour être un volume moindre. En particulier, lorsque le volume de contrôle Vc est adjacent au volume de référence Vref, on choisit Vc nettement plus petite que Vref. Lorsque différents, le volume de contrôle Vc peut être en aval du volume de référence Vref, de sorte que le contenu du volume de contrôle Vc soit connu grâce au volume de référence Vref.

Une fois le débit obtenu, le capteur 10 peut générer une consigne d'injection pour la pompe 32 (étape E5). Le fluide injecté par l'injecteur 20 est du fluide qui se trouvait dans le volume de référence Vref lorsque ce dernier a été déterminé comme étant empli. La consigne d'injection est configurée pour injecter un volume moindre que le volume de référence Vref. La consigne d'injection est générée à l'aide du débit et d'une consigne prédéterminée, la consigne prédéterminée correspondant à une quantité désirée de volume injecté. Cette consigne d'injection présente un intérêt supérieur lorsque le capteur 10 est incorporé à un injecteur 20. Plus de détails seront donnés par la suite.

Cette méthode de calcul de débit permet de s'affranchir de nombreuses limitations : il importe désormais peu que la pompe puisse être modifiée ou changée, que la pompe vieillisse et que ses caractéristiques changent, que le fluide soit mixte avec liquide et gaz ou plusieurs liquides différents. Le calcul local du débit, le cas échéant avec une injection qui se fait dans une plage de fonctionnement très proche de celle du calcul du débit, permet une rétroaction sur la pompe et surmonte toutes ces difficultés. La répétabilité de l'injection s'en trouve fortement améliorée.

De plus, le capteur 10 et l'injecteur 20 permettent d'obtenir des informations sur le débit en utilisant peu de fluide mixte. Dans certaines applications, la quantité disponibles de fluide mixte peut être faible et le sacrifice de microlitres peut poser des difficultés pour l'injection subséquente.

En outre, comme les sondes 14, 16 peuvent être proches (moins de 20mm, ou même moins de 10mm), le capteur 10 et l'injecteur 20 peuvent être compact. Les dimensions maximales de l'injecteur 20 peuvent être inférieurs à 5cm, voire 4cm.

### Mode de réalisation en absolu

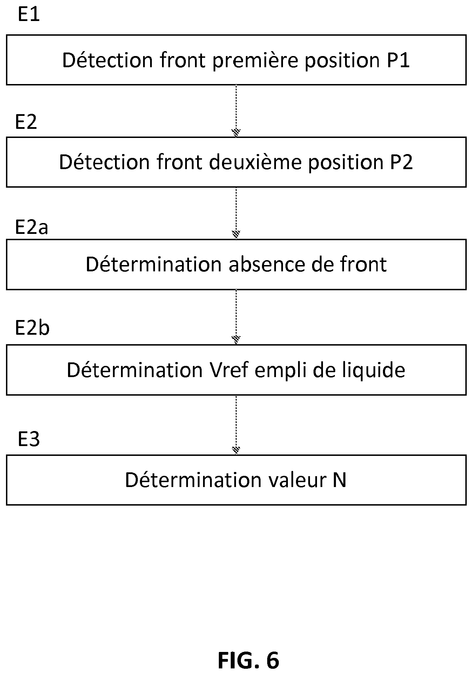

Dans le premier mode de réalisation, dit « absolu », illustré sur les figures 1, 5 et 6, le volume de référence Vref est défini par la première position P1 et la deuxième position P2, c'est-à-dire entre les deux sondes 14, 16. Le capteur 10 utilise donc la détection de changement de phase par la première sonde 14 et par la deuxième sonde 16 pour déterminer que le volume de référence Vref est empli de liquide ou de liquide homogène. La distance entre P1 et P2 peut être comprise entre 3 et 20 mm, ou entre 2 et 8mm (par exemple 5mm).

L'ECU met alors en oeuvre une étape E2a de détermination que la première sonde 14, après avoir détecté le changement de phase à l'étape E1 et avant que la deuxième sonde 16 ne détecte le changement de phase, ne détecte pas d'autre changement de phase. L'étape E2a peut être effectuée une fois l'étape E2 de détection de changement de phase par la deuxième sonde 16 effectuée (vérification *a posteriori).* En effet, si la première sonde 14 détecte un premier changement de phase puis un deuxième changement de phase avant que le premier changement de phase ne soit détecté par la deuxième sonde 16, cela signifie qu'entre la première position P1 et la deuxième position P2 (c'est-à-dire dans le volume de référence Vref) se trouve une juxtaposition de liquide/gaz ou de deux liquides non homogènes. Formulé différemment, il faut que la première sonde ne détecte pas d'autre changement de phase avant que la deuxième sonde ne détecte le changement de phase détecté par la première sonde à l'étape E1, ce qui permet d'avoir un front continu entre la première sonde 14 et la deuxième sonde 16. Par exemple, si, après traitement des données par l'ECU, les sondes 14, 16 génèrent un 1 pour du liquide et un 0 pour du gaz, il faut que la sonde 14 ne génère que du 1 jusqu'à tant que la sonde 16 passe de 0 à 1. En réponse à l'étape E2a, l'ECU met en œuvre une étape E2b de détermination que le volume de référence Vref est empli de liquide.

Dans ce mode de réalisation, le volume de contrôle Vc est le même que le volume de référence Vref (pas seulement la même valeur, mais le même espace du conduit 12). Cela signifie que le même volume physique du conduit 12 est utilisé pour déterminer qu'une quantité suffisante de liquide (le cas échéant de même liquide) est présente et pour déterminer la vitesse de déplacement du liquide.

Dans ce mode de réalisation, les sondes 14, 16 peuvent mesurer à une position donnée un changement de changement de phase du fluide mixte indépendamment de l'état du fluide mixte en amont et en aval de ladite position (d'où l'appellation du mode de réalisation en « absolu »). Par exemple, les sondes 14, 16 peuvent comprendre une sonde optique avec une source de lumière et un capteur optique, de sorte que la source de lumière traverse le conduit 12 ; un changement de changement de phase étant associé à un changement de fluide et donc généralement un changement de propriété optique (réfraction, absorbance, etc.), le capteur optique va recevoir un signal lumineux différent. L'optique présente l'avantage de ne pas pénétrer dans le conduit 12 et de ne pas interagir chimiquement avec le fluide mixte. Il est nécessaire en revanche que le conduit soit adapté pour laisser passer la lumière (matériau transparent à la longueur d'onde émise par le capteur optique ou zone de mise en place spécifique). Par exemple, les sondes peuvent comprendre une sonde à électrodes, avec un couple d'électrodes rapprochées à l'intérieur du conduit 12 ; un changement de changement de phase étant associé à un changement de fluide et donc généralement un changement de propriété électrique (conductivité, etc.). Par exemple, les sondes peuvent comprendre une sonde thermique ; une résistance chauffe localement le fluide et un capteur de température situé par exemple à une même position le long du conduit 12 récupère la donnée de température, qui dépend de la nature du fluide. Par exemple, les sondes peuvent comprendre une sonde capacitive. Préférablement, les deux sondes 14, 16 sont de même nature mais des combinaisons sont possibles.

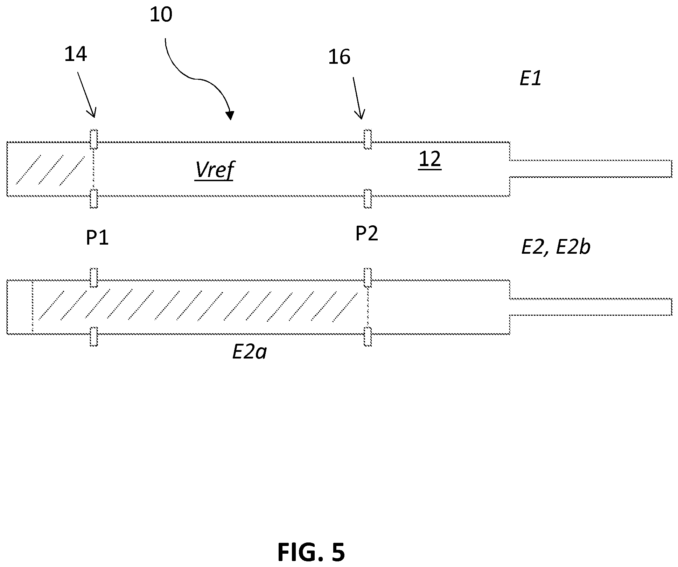

La figure 5 illustre le capteur 10 à l'étape E1, quand un changement de phase atteint la première sonde 14 et à l'étape E2, quand un changement de phase atteint la deuxième sonde 16 et aux étapes E2a et E2b. Comme le volume de référence Vref était vide au moment de l'étape E0, l'ECU est assurée que le changement de phase détecté par la deuxième sonde 16 a été détecté par la première sonde 14. En comptant les changements de phase, l'ECU peut établir une correspondance de changements de phase détectés par la première sonde 14 et la deuxième sonde 16. Les illustrations de la figure 5 montrent le cas de l'étape E2a, où l'on voit que le fluide qui occupe le volume de référence Vref n'est que du liquide (pas de gaz).

### Mode de réalisation en relatif (non couverts par les revendications)

Dans le deuxième mode de réalisation, illustré sur les figures 2 et 6 à 11, le volume de référence Vref est défini notamment par la première position P1. En particulier, la première sonde 14 comprend une paire d'électrodes 14a, 14b (« première paire d'électrodes ») connectées électriquement entre elles en circuit ouvert et disposées espacées entre elle le long du conduit 12. Les électrodes 14a, 14b s'étendent au moins partiellement à l'intérieur du conduit 12 pour être en contact avec le fluide mixte et fermer, ou non, le circuit. La source d'énergie 19 et l'ECU sont configurées pour appliquer une différence de potentiel entre les deux électrodes 14a, 14b.

La première paire d'électrodes comprend une première électrode amont 14a et une première électrodes aval 14b (amont et aval se référant à la circulation forcée du fluide dans le capteur 10, donc sur les figures à la gauche et la droite), qui est espacée le long du conduit 12 de la première électrode amont 14a.

Les deux électrodes de la paire d'électrodes 14a, 14b de la première sonde 14 définissent entre elles le volume de référence Vref. Lorsqu'un fluide empli entièrement le volume de référence Vref, la résistivité chute et un courant s'établit entre la paire d'électrodes 14a, 14b, donc la tension entre les électrodes 14a, 14b chute. Cela signifie qu'un changement de phase est identifié au niveau de la première électrode aval 14b. Après traitement des données par l'ECU, la sonde 14 peut générer un 1 et un 0 en fonction respectivement de présence de liquide ou de gaz. Dans ce mode de réalisation, la première position P1 correspond à la position de la première électrode aval 14b puisque c'est à son niveau que le changement de changement de phase est identifié. Par conséquent, l'ECU peut mettre en œuvre une étape E1a, en réponse à l'étape E1, de détermination que le volume de référence Vref est empli de liquide uniquement.

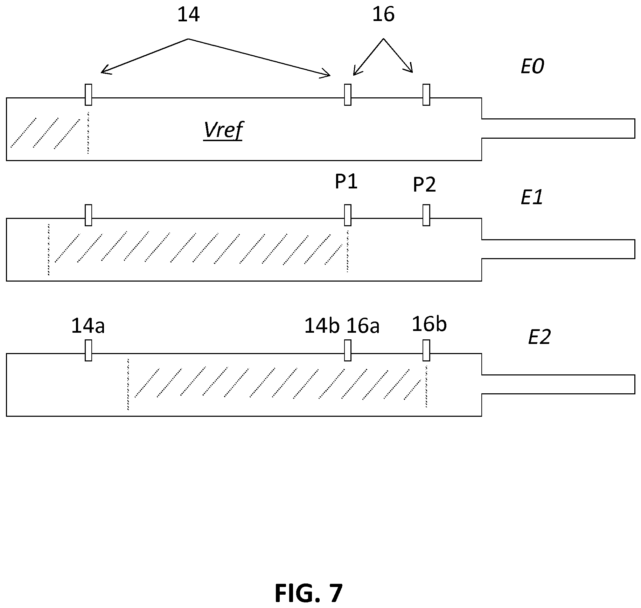

La paire d'électrodes permet aussi de détecter directement que le volume de référence Vref est empli de liquide, sans nécessairement détecter de changement de phase : par définition du volume de référence Vref (entre les deux électrodes 14a, 14b), dès lors que la différence de potentiel entre la première paire 14a, 14b est faible, l'ECU sait que le volume de référence Vref est empli de liquide. La figure 7 illustre l'étape E0 et l'étape E1.

Néanmoins, comme le conduit 12 est vide ou quasiment vide avant l'étape préliminaire E0, la différence de potentiel entre les deux électrodes 14a, 14b est, au moment de l'étape E0, élevée, de sorte que de l'identification que le volume de référence Vref est empli de liquide s'accompagne d'un changement de changement de phase.

Si le fluide mixte forme une alternance de fronts avec un volume inférieur au volume de référence Vref, il n'y aura pas de continuité électrique dans le fluide dans le volume de référence Vref, si bien que la première sonde 14, avec ses deux électrodes 14a, 14b, ne détectera pas nécessairement de changement de phase. Dès que le potentiel chute en revanche, l'ECU est assurée que le volume de référence Vref est empli de liquide.

La deuxième sonde 16 peut aussi comprendre une paire d'électrodes 16a, 16b (« deuxième paire d'électrodes »), connectées électriquement entre elles en circuit ouvert et disposées entre elle le long du conduit 12. Les électrodes 16a, 16b s'étendent au moins partiellement à l'intérieur du conduit 12 pour être en contact avec le fluide mixte et fermer, ou non, le circuit. La source d'énergie 19 et l'ECU sont configurées pour appliquer une différence de potentiel entre les deux électrodes 16a, 16b.

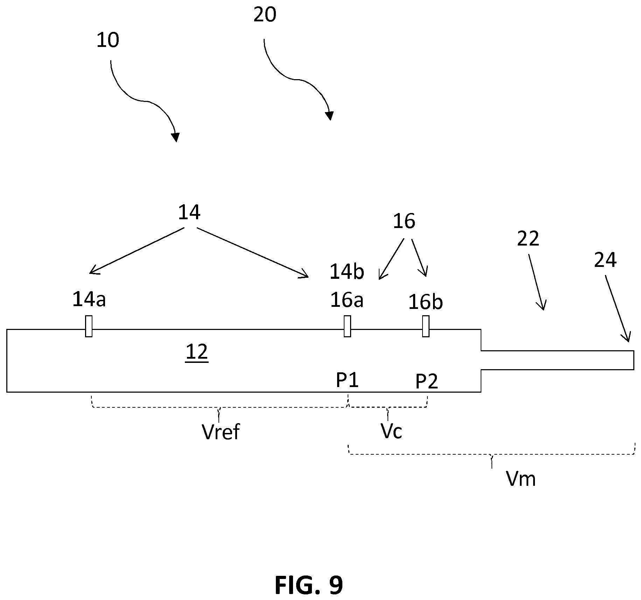

La deuxième paire d'électrodes comprend une deuxième électrode amont 16a et une deuxième électrodes aval 16b (amont et aval se référant à la circulation forcée du fluide dans le capteur 10, donc gauche et droite sur les figures), qui est espacée le long du conduit 12 de la deuxième électrode amont 16a. La deuxième paire d'électrode 16a, 16b est différente de la première paire d'électrode 14a, 14b (au moins une électrode différente). Une électrode peut être en commun, afin de gagner en compacité et de minimiser le nombre d'éléments à monter sur le conduit 12. Les figures 9 à 11 illustrent différentes configurations.

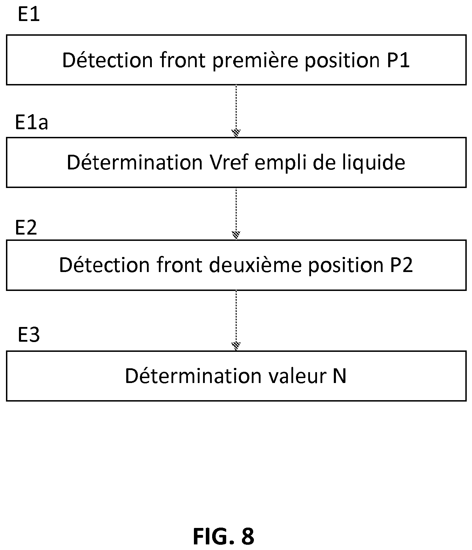

La deuxième paire d'électrode 16a, 16b fonctionne techniquement comme la première paire d'électrodes 14a, 14b. La position P2 est définie par la deuxième électrode aval 16b, parce que la deuxième paire d'électrodes détecte effectivement un changement de phase à la position P2. Par conséquent, le capteur 10 comprend deux sondes 14, 16 qui peuvent détecter un changement de phase. Dans le mode de réalisation de la figure 7, la première paire d'électrodes détecte un changement de phase, ce qui signifie que le volume de référence Vref est empli de liquide (étape E1 et E1a des figures 7 et 8). Le fluide se déplace ensuite jusqu'à la deuxième électrode amont 16a (qui correspond ici à la première électrode aval 14b) puis la deuxième électrode aval 16b (étape E2 des figures 7 et 8). Le volume de contrôle Vc est défini entre la première position P1 et la deuxième position P2 (donc entre la première électrode aval 14b et la deuxième électrode aval 16b). Le volume de contrôle Vc est connu et sa valeur est stockée par l'ECU. Avec la valeur N de la grandeur physique de déplacement (obtenue à l'étape E3), qui correspond typiquement au temps écoulé ou au nombre de coups de pompe pour que le fluide passe de la première position P1 à la deuxième position P2 (c'est-à-dire traverse le volume de contrôle Vc), l'ECU peut déterminer le débit à l'étape E4.

Dans ce mode de réalisation, le volume de contrôle Vc peut être adjacent au volume de référence Vref (voir figure 2). Le volume de contrôle Vc est choisi pour être plus petit que le volume de référence Vref, pour qu'il n'y ait pas d'incertitude quant à son remplissage par du liquide (ou du liquide homogène).

Pour simplifier les opérations, la deuxième position P2 est choisie pour être en aval de la première position P1. Dit autrement, la deuxième électrode aval 16b est en aval de la première électrode aval 14b. De la sorte, le compteur ne se déclenche qu'après l'étape E1a (une fois que le volume de référence Vref est empli de liquide).

En particulier, dans le mode de réalisation illustré en figure 9, la première électrode aval 14b est confondue (ou commune) avec la deuxième électrode amont 16a. L'agencement le long du conduit 12 peut donc être dans l'ordre suivant : première électrode amont 14a, première électrode aval 14b (qui fait office de deuxième électrode amont 16a), deuxième électrode aval 16b. Le volume de contrôle Vc est donc adjacent à, et en aval du, volume de référence Vref. Dès lors que le volume de référence Vref est empli de liquide, l'ECU est assuré que le volume de contrôle Vc pourra être empli de liquide (condition nécessaire pour détecter le front avec la deuxième paire d'électrode 16a, 16b). Les électrodes 14a, 14b/16a, 16b peuvent être, respectivement et dans l'ordre : positive, négative, positive ou bien négative, positive, négative.

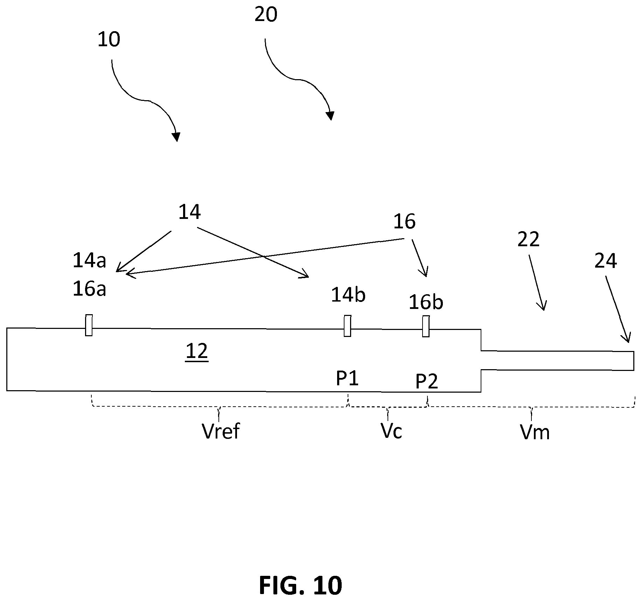

Alternativement, comme illustré en figure 10, la première électrode amont 14a est confondue (ou commune) avec la deuxième électrode amont 16a. L'agencement le long du conduit 12 peut donc être dans l'ordre suivant : première électrode amont 14a (qui fait office de deuxième électrode amont 16a), première électrode aval 14b, deuxième électrode aval 16b. Dans cette alternative, pour que la deuxième sonde 16 détecte le changement de phase, il faut que le volume de référence Vref et le volume de contrôle Vc soit empli de liquide, ce qui est une contrainte additionnelle. En revanche, la précision des mesures peut être améliorée du fait des distances qui sont plus grandes. Les électrodes 14a/16a, 14b, 16b peuvent être, respectivement et dans cet ordre : positive, négative, négative ou bien négative, positive, positive.

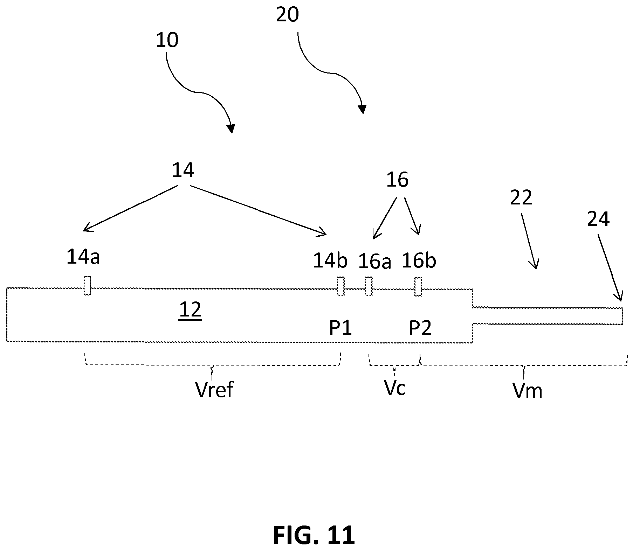

Alternativement, comme illustré en figure 11, la deuxième paire 16a, 16b est entièrement distincte de la première paire 14a, 14b. Elle peut alors se trouver de part et d'autre de la première électrode aval 14b ou en aval de la première électrode aval 14b (comme illustré). Cette variante ne mutualise toutefois pas les électrodes et, comme dans le cas illustré en figure 11, peut générer un volume intermédiaire, entre le volume de référence Vref et le volume de contrôle Vc, dont il faut tenir compte lors de la conception pour la consigne d'injection de l'étape E5.

La pompe 32 peut être pilotée en débit élevé jusqu'à l'obtention du volume de référence Vref (étape E1a) et en débit faible jusqu'à la détection du changement de phase à la deuxième position (étape E2) ou bien l'obtention de la valeur N de la grandeur physique de déplacement (étape E3) et/ou du débit (étape E4). L'intérêt du pilotage en débit élevé est de ne pas créer de goulot d'étranglement au niveau d'un captation de fluide dans le circuit fluidique 30. De plus, cela permet d'amorcer la pompe (en l'immergeant). La pompe 32 est toutefois piloté sous le même régime pour le déplacement la position P1 et la position P2 (étapes E2, E3) et pour la génération de la consigne d'injection (E5). Ce pilotage trouve particulièrement application dans le mode de réalisation des figures 9 et 10, où le calcul du débit se fait après que le volume de référence Vref a été complètement empli de liquide ou de liquide homogène.

Les électrodes 14a, 14b, 16a, 16b pénètrent à l'intérieur du conduit 12 (des ouvertures sont alors prévues dans le conduit) afin de pouvoir être en contact avec le fluide mixte. Les sondes 14, 16 sont respectivement reliées à l'ECU et à la source d'énergie 19 pour appliquer la différence de potentiel sur les électrodes.

Les distances entre électrodes peuvent être comprises entre 3 et 20mm. Par exemple, l'espacement des électrodes de la première paire peut être de 8mm et ceux de la deuxième paire de 5mm. Dit autrement, le volume de référence Vref a une longueur de 8mm et le volume de contrôle Vc de 5mm.

### L'injecteur 20

La figure 1 représente (partiellement) un injecteur 20 incorporant un capteur 10, avec la buse d'injection 22 et son extrémité distale d'injection 24 ; la figure 3 représente l'injecteur 20 dans un circuit fluidique 30 avec la pompe 32.

L'injecteur 22 présente un volume mort Vm, qui correspond à un volume du conduit 12 et/ou un volume de la buse d'injection 22 que le fluide doit parcourir depuis la deuxième position P2 jusqu'à l'extrémité distale d'injection 24. Afin de s'assurer que le débit calculé avec le capteur 10 soit considéré comme stable pour l'injection, il est préférable que le volume mort Vm soit faible. Dans un mode de réalisation, l'injecteur 20 est conçu pour que le volume mort Vm soit plus faible que le volume de référence Vref. On s'assure ainsi que la plage de fonctionnement de la pompe 32, lorsqu'elle pompe pour injecter, reste dans la plage de fonctionnement de la pompe 32 pour l'obtention du débit. Il est supposé qu'à T+deltaT, le débit ne varie que de D+deltaD, et que si deltaT est faible, deltaD est faible aussi. Dans le cas du mode de réalisation dit « relatif », l'injecteur 20 peut être conçu pour que le volume mort Vm soit plus faible que le volume de référence Vref additionné au volume de contrôle Vc.

Dans le cas du circuit fluidique de la figure 3, pour la génération de la consigne d'injection (étape E5), l'ECU stocke la valeur du volume mort Vm. Avec cette valeur du volume mort Vm et une consigne prédéterminée, l'ECU peut calculer une consigne d'activation à destination de la pompe 32.

Le procédé peut alors comprendre les sous-étapes suivantes pour la génération de la consigne d'injection (étape E5). En particulier, l'étape E5 peut se fait selon deux modes de réalisation.

Dans un premier mode de réalisation, dit direct, l'ECU cherche à injecter directement la quantité souhaitée de fluide. La pompe 32 a soit été arrêtée à l'étape E2, soit le calcul se fait en temps réel et il n'y a pas eu besoin d'arrêter la pompe : le front du liquide qui a empli le volume de référence Vref se trouve à la position P2. De la position P2 à l'extrémité distale 24 de l'injecteur 20 se trouve le volume mort Vm. Par conséquent, l'ECU calcule une consigne d'injection pour la pompe 32 qui tient compte du volume d'injection désiré (consigne prédéterminée), du volume mort Vm et du débit déterminé de la pompe 32. Formellement, si on note D le débit, Vm la valeur du volume mort (par abus de langage) et V le valeur de la consigne prédéterminée, la consigne d'activation Nact de la pompe 32 (en coup de pompe ou en durée) de Nact = (V+Vm)/D.

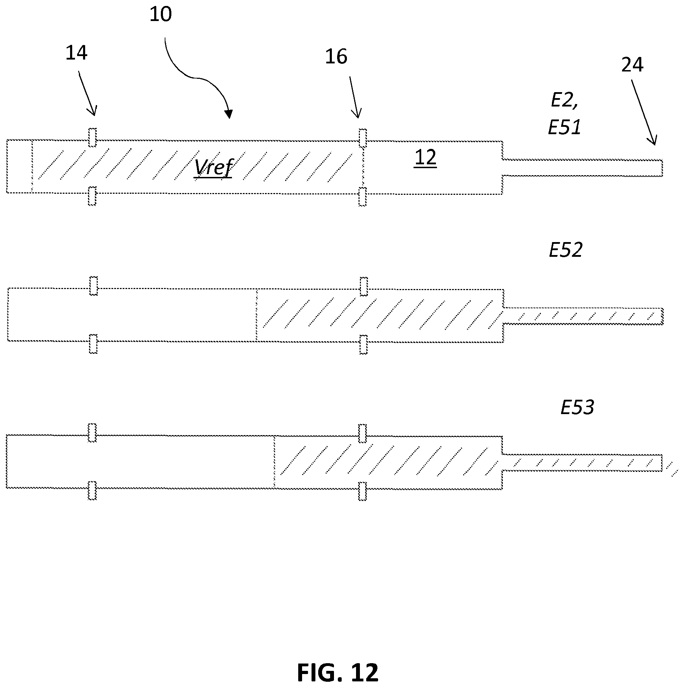

Dans un deuxième mode de réalisation, dit à précharge, illustré en figure 12, l'ECU précharge l'injecteur 20 avec le fluide, pour ensuite effectuer l'injection ultérieurement. L'ECU calcule une consigne de précharge pour la pompe 32 (étape E51), qui tient compte du volume mort Vm et du débit déterminé de la pompe 32. Le but de la précharge est d'amener du fluide issu du volume de référence Vref au niveau de l'extrémité distale pour qu'ensuite l'injection soit immédiate et se fasse directement avec le volume souhaité. En particulier, la précharge peut consister à activer la pompe 32 pour amener le front au niveau de l'extrémité distale 24 (étape E52), sans versement ou bien avec un léger versement pour sacrifier une portion du liquide afin d'être certain que la précharge soit faite (étape E53). Dit autrement, l'ECU génère une consigne de précharge pour la pompe 32 d'un volume égal au volume Vm ou égal à quelques pourcents en plus du volume mort, de type 1+A%Vm, A étant compris entre 0 et 50 par exemple (voir 25). Si le volume mort Vm fait 3µL, le volume injecté pour la pré-charge peut être de 8µL (sacrifice de 5µL). Pour permettre la précharge, l'injecteur 20 est préférablement dans une position de purge (et non pas en regard de l'élément sur lequel l'injection doit être effectuée). La précharge permet à l'injecteur 20 d'être prêt à injecter lors d'une phase ultérieure d'injection.

Le débit est avantageusement calculé directement dans l'unité de la consigne de la pompe 32. Par exemple, si la pompe 32 se commande en temps d'activation (en seconde par exemple) ou en coups de pompe, le débit déterminé à l'étape E4 se fait dans la même unité. Cela évite de mettre en œuvre une conversion qui nécessite de connaître d'autres caractéristiques de la pompe et qui sont susceptibles d'être imprécises ou d'évoluer avec le temps.

Dans un mode de réalisation, le capteur 10 et/ou l'injecteur 20 est utilisé comme débulleur, en injectant sélectivement le liquide (ou le liquide homogène) dans un réservoir de liquide et le gaz dans un réservoir de gaz (ou l'air libre). Par exemple, l'injecteur 20 peut être mobile en translation ou rotation pour sélectivement déverser le fluide vers les deux réservoirs précités (ou les réservoirs sont mobiles et l'injecteur 20 fixe). La commande du mouvement peut être faite par l'ECU qui connait le contenu du volume de référence Vref grâce aux sondes 14, 16.

Le capteur 10 et/ou l'injecteur 20 et/ou le circuit fluidique 30 trouve une application dans un dispositif d'analyse d'urine 100 et plus particulièrement dans une station 200 pour disposition d'analyse d'urine, la station 200 recevant une cartouche 204 comprenant des réactifs à l'urine.

### Station et dispositif d'analyse d'urine

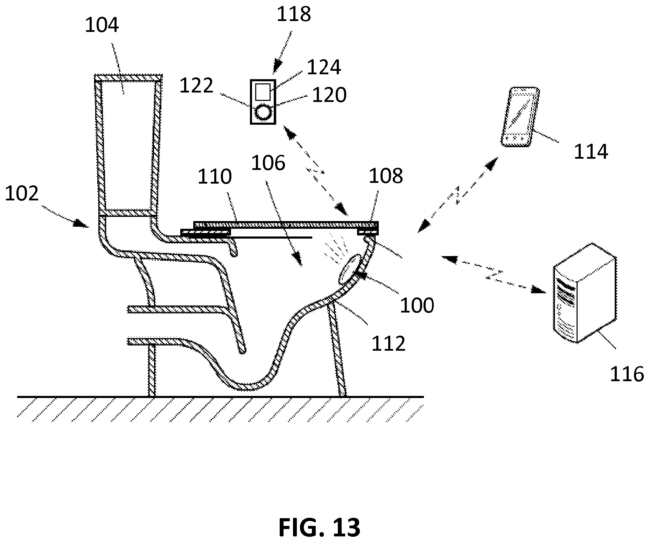

La figure 13 illustre schématiquement un dispositif d'analyse d'urine 100 monté sur des toilettes 102. De manière connue, les toilettes 102 comportent un réservoir d'eau 104, une cuvette 106, un siège 108 et un couvercle 110. Le dispositif d'analyse d'urine 100 est agencé sur une paroi interne 112 de la cuvette 106 des toilettes. Avantageusement, le dispositif d'analyse d'urine 100 est entièrement reçu dans la cuvette des toilettes, ce qui lui permet d'être discret.

Le dispositif d'analyse d'urine 100 peut être positionné sur la trajectoire d'un jet d'urine secrété par un utilisateur. Le dispositif d'analyse d'urine 100 reçoit un jet d'urine lorsqu'un utilisateur urine en position assise dans les toilettes. La position du dispositif d'analyse d'urine est alors adaptée pour tout type d'utilisateur, de sexe masculin ou féminin, quel que soit son âge. L'utilisateur peut alors uriner dans les toilettes sans se préoccuper de la position du dispositif d'analyse d'urine.

Le dispositif d'analyse d'urine 100 peut être également positionné sur la trajectoire d'une chasse d'eau provenant du réservoir 104. Le dispositif d'analyse d'urine 100 peut ainsi être rincé lors de l'actionnement de la chasse d'eau. Le dispositif d'analyse d'urine 100 est hygiénique.

Le dispositif d'analyse d'urine 100 peut communiquer avec un terminal mobile 114 (type smartphone) et/ou un serveur externe 116. Dans un mode de réalisation, le dispositif d'analyse d'urine 100 communique avec le terminal mobile 114 (par exemple directement via Bluetooth comme du Bluetooth Low Energy) et le terminal mobile 114 communique avec le serveur 116 (via une connexion cellulaire ou WiFi). Dans un autre mode de réalisation, le dispositif d'analyse d'urine 100 peut communiquer directement avec le serveur 116 par un réseau cellulaire.

Pour lancer une mesure par le dispositif d'analyse d'urine 100, un activateur externe 118 peut être prévu. L'activateur externe 118 peut comprendre un bouton 120 et/ou un capteur biométrique 122. Un écran 124 peut être installé sur l'activateur externe 118 pour afficher les données obtenues par le dispositif d'analyse d'urine 100. Ce dernier et l'activateur externe 118 communiquent de façon sans-fil.

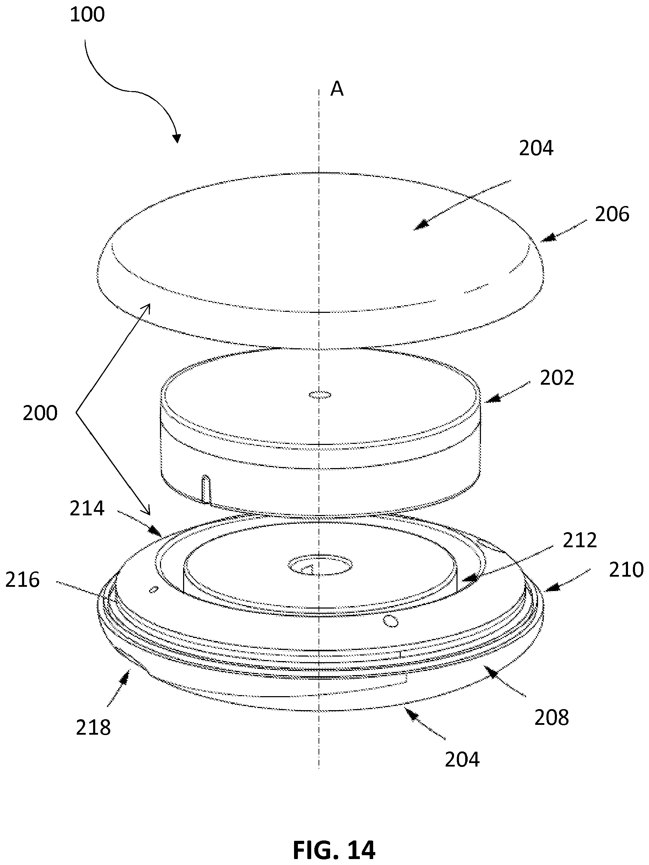

Comme illustré sur la vue éclatée de la figure 14, le dispositif d'urine 100 comprend une station 200 et une cartouche 202, montée amovible dans la station 200. La station 200 comprend notamment un boitier 204 qui est, selon une réalisation particulière, formé comme un assemblage de deux demi-coques : une coque avant 206 et d'une coque arrière 208. La coque avant et la coque arrière forment une jointure 210 du boitier, dans un plan normal à l'axe A. L'assemblage du dispositif d'analyse d'urine est facilité lorsque le boitier est constitué de la coque avant et de la coque arrière. Le boitier 204 renferme un ensemble de test. L'ensemble de test est destiné à analyser l'urine étant reçue dans le dispositif d'analyse d'urine 100. La station 200 comporte en outre un logement annulaire 212, à l'intérieur du boitier 204, agencé autour d'un axe de rotation A. Le logement annulaire 212 est configuré pour recevoir au moins partiellement la cartouche 202 montée à rotation autour de l'axe de rotation A (une fois en position dans le logement annulaire 212). La cartouche 202 comprend une pluralité de supports de test intégrant un réactif, par exemple un réactif sec agencés le long d'un cercle ou d'un arc de cercle autour de l'axe de rotation A. Dans un mode de réalisation et pour la suite de la description, les supports de tests sont des bandelettes de test. En particulier, le logement annulaire 212 peut être partiellement délimité, avec jeu fonctionnel, par un couvercle interne 214, monté par exemple avec la coque arrière 208, pour protéger des composants de l'ensemble de test. Le couvercle interne 214 peut comprendre une portion radiale externe, pour protéger les composants radialement externes au logement annulaire 212 et une portion radiale interne, pour protéger les composants radialement internes au logement annulaire 212.

Le logement annulaire 212 s'étend typiquement sur 360° et forme une gorge configurée pour recevoir en partie la cartouche 202.

Dans la présente description, la locution « radialement interne au logement annulaire signifie plus proche de l'axe de rotation A que le logement annulaire n'est proche de l'axe de rotation A. De façon similaire, la locution « radialement externe au logement annulaire signifie « plus éloigné de l'axe de rotation A que le logement annulaire n'est éloigné de l'axe de rotation A ».

Le logement annulaire 212 est accessible par exemple en détachant la coque avant 206 de la coque arrière 208. Les coques avant 206 et arrière 208 peuvent être vissées l'une à l'autre à l'aide d'un filetage 216.

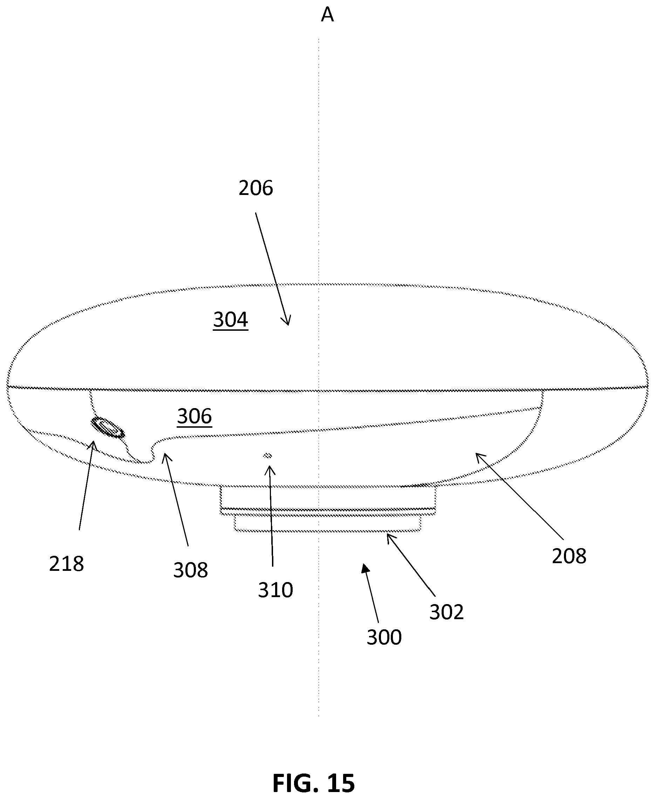

La station 200, en particulier le boitier 204, comprend en outre un orifice de collecte 218, positionnée par exemple sur la coque arrière 208 sur la figure 15. L'orifice de collecte 218 peut recevoir l'urine ruisselant par gravité sur la surface extérieure du boitier 204. Plus de détails sur cet orifice de collecte 218 seront donnés par la suite.

Le boitier 204 est agencé dans la cuvette 106 des toilettes de manière amovible. Le dispositif d'analyse 100 peut alors être retiré ou repositionné dans les toilettes. En outre, le dispositif d'analyse d'urine 100 ou le boitier 204 peut être retiré pour recharger une batterie ou pour changer la cartouche 202.

Dans l'exemple illustré à la figure 13, le boitier 204 est agencé sur la paroi interne 112 des toilettes. Le boitier 204 est positionné par un élément de fixation 300, dont un mode de réalisation est visible notamment sur la figure 15. L'élément de fixation 300 peut comporter des aimants 302 et/ou une surface adhésive/à ventouse qui peut coopérer Cette configuration permet de facilement retirer ou repositionner le boitier dans les toilettes. Dans une autre configuration, le dispositif d'analyse d'urine 100 comprend un crochet solidaire à une extrémité du boitier 204 et configuré pour s'attacher à l'autre extrémité à un rebord de la cuvette 106 (sous le siège 108 par exemple).

La station 200, en particulier le boitier 204, comprend en outre un orifice de vidange 310, positionnée sur la coque arrière 208 sur la figure 15. L'orifice de collecte 218 peut recevoir l'urine ruisselant par gravité sur la surface extérieure du boitier 204 et l'orifice de vidange 310 permet de vidanger les différents fluides captés par le dispositif 100. Plus de détails sur cet orifice de vidange 310 seront donnés par la suite.

### Caractéristiques générales du boitier

Comme représenté sur la figure 15, le boitier 204 a une forme extérieure de galet circulaire. En d'autres termes, le boitier a une forme de sphéroïde aplatie. L'axe de rotation A est l'axe médian du boitier. Le boitier comporte une face avant 304 et une face arrière 306, sensiblement normales à l'axe A. La face avant 304 comprend typiquement la surface externe de la coque avant 206 et la face arrière 306 comprend notamment la surface externe de la coque arrière 208. Ainsi, une collecte d'urine peut se faire directement depuis les faces 304, 306 du boitier. Le boitier 204 sert de collecteur d'urine.

La face avant 304 est orientée vers l'intérieur de la cuvette 106. La face avant 304 est alors destinée à recevoir de l'urine lorsque l'utilisateur urine en position assise sur les toilettes. La face arrière 306 est orientée face à la paroi intérieure 112 de la cuvette 106. La face avant 304 et la face arrière 306 sont reliées par des bords courbés 308. Alors, la surface extérieure du boitier 204, constituée de la face avant 304, la face arrière 306 et les bords courbés 308, est définie par des lignes courbes, formant un objet généralement convexe. Le boitier est par exemple dépourvu d'arêtes. De l'urine peut ruisseler sur l'ensemble de la surface extérieure du boitier sans décrocher du boitier ou former de bulles d'air, pouvant compromettre une analyse de l'urine. La demande PCT/EP2021/055377 décrit en détail la forme du boitier 204 pour permettre une captation efficace de l'urine.

Dans un mode de réalisation, le boitier 204 a un diamètre, mesuré dans la direction normale à l'axe A, compris entre 50 mm et 150 mm, par exemple voisin de 100 mm. Le boitier 204 a également une épaisseur, mesuré selon la direction de l'axe A, comprise entre 15 mm et 50 mm, par exemple voisine de 30 mm. Ainsi, le boitier est suffisamment compact pour être entièrement reçu dans la cuvette des toilettes. Le dispositif d'analyse d'urine est discret. En outre, le boitier est suffisamment étendu pour systématiquement entrer en contact avec de l'urine étant reçu dans la cuvette. L'utilisateur peut alors uriner dans les toilettes sans se soucier du dispositif d'analyse d'urine, ou à défaut viser sommairement.

La coque avant 206 et la coque arrière 208 sont assemblées pour maintenir la surface extérieure du boitier définie par des lignes courbes.

Dans un mode de réalisation non illustré, la coque avant 206 ou la coque arrière 208 comprend un capot amovible (étanche) permettant le remplacement de la cartouche 202. Plutôt que de démonter la coque avant 206 pour accéder au logement annulaire 212, il suffit alors d'enlever le capot amovible. Dans l'exemple illustré, le capot amovible est formé par la coque avant 206 du boitier 204. Le capot amovible peut alors être retiré par dévissage de la coque avant 206 par rapport à la coque arrière 208. Le boitier 204 comporte moins de jointures pouvant être salies et/ou infiltrées par l'eau des toilettes.

Le boitier 204 présente un orifice de collecte 218, déjà présenté en relation avec la figure 15. L'orifice de collecte 218 peut recevoir l'urine ruisselant par gravité sur la surface extérieure du boitier. Une collecte d'urine est réalisée directement depuis les faces 305, 306 du boitier. L'orifice de collecte 218 est situé sur une extrémité inférieure 402 du boitier 204. L'extrémité inférieure 404 est orientée vers le fond de la cuvette 106 lorsque le boitier 204 est positionné dans la cuvette 106 des toilettes. Cette position correspond à une position normale d'utilisation. Cette position permet une collecte de l'urine ruisselant par gravité sur la majorité de la surface extérieure du boitier.

En l'espèce, une distance D séparant l'orifice de collecte 218 d'une bordure inférieure 404 du boitier est inférieure à 40 mm, par exemple inférieure à 20mm. Selon une réalisation particulière, l'orifice de collecte 218 est agencé à quelques millimètres au-dessus de la bordure inférieure du boîtier. En variante, l'orifice de collecte peut être sur la bordure inférieure 404.

L'orifice de collecte 218 est une ouverture circulaire, de diamètre par exemple compris entre 0,3 mm et 2 mm. Le diamètre de l'orifice de collecte peut être choisi afin de maximiser le volume d'urine collecté de la surface extérieure du boitier.

Le boitier 204 présente un orifice de vidange 310, déjà présenté en relation avec la figure 15. L'orifice de vidange 310 permet de purger le dispositif d'analyse d'urine 100 d'un excès d'urine.

Une cartouche et un ensemble de test permettant d'exploiter des supports de test de la cartouche, comme bandelettes (par exemple des bandelettes colorimétriques ou des *lateral flow),* vont être décrit (plus de détails en fin de description). Les bandelettes sont ici également désignées « bandelettes de tests » ou plus simplement « bandelette ». L'ensemble de test comprend notamment un injecteur et un analyseur (par exemple un analyseur optique). La cartouche 202 est entrainée en rotation dans le boitier 204 par un actionneur d'entrainement, positionné dans le boitier 204, de sorte que les bandelettes de test peuvent défiler successivement devant l'injecteur 604 et l'analyseur 606. L'ensemble de test comprend également un capteur de position 624, pour identifier une position des bandelettes dans le boitier.

L'injecteur permet d'injecter un volume contrôlé d'urine sur une bandelette lorsque ladite bandelette est dans une zone d'injection du logement annulaire 212. L'analyseur permet d'analyser une bandelette lorsque ladite bandelette est dans une zone d'analyse du logement annulaire 212. Le capteur de position permet d'obtenir une position de la cartouche ou de la bandelette lorsqu'un marqueur associé à celle-ci se trouve dans une zone de contrôle du logement annulaire 212.

En particulier, l'analyseur peut détecter un changement de couleur du support de test, qui peut être une bandelette (analyse colorimétrique). L'analyseur peut alors être un analyseur optique, avec une source de lumière et un capteur optique.

### Cartouche

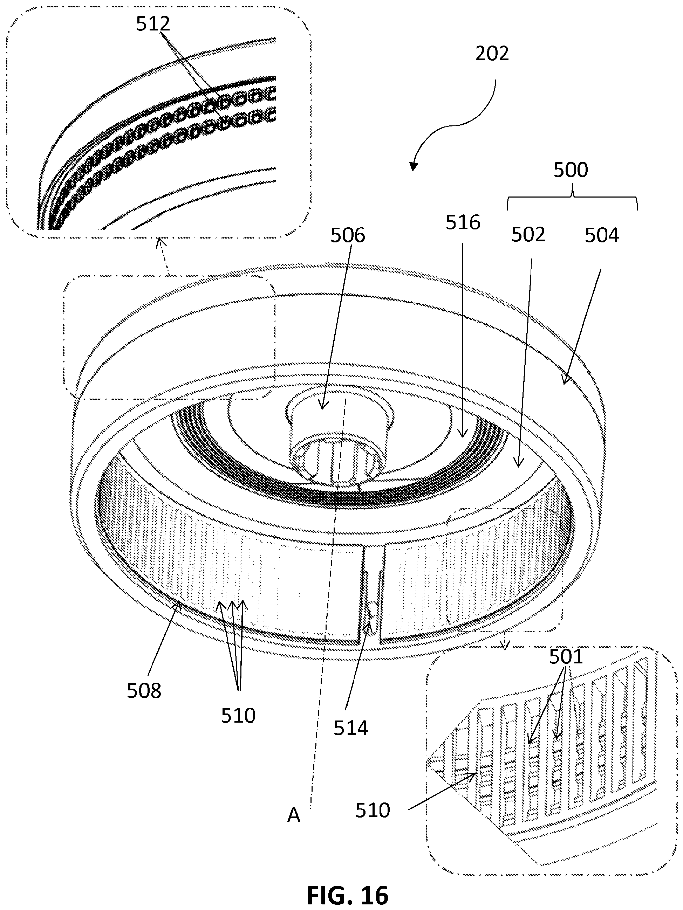

La figure 16 illustre une vue éclatée d'un mode de réalisation de la cartouche 202. La cartouche 202 intègre des supports de tests destinées à recevoir de l'urine lorsque la cartouche 202 est montée dans la station 200 et, en particulier, dans le logement annulaire 212. Le support de test comprend un réactif qui réagit une fois en contact de l'urine. Comme illustrés sur la figure 16, les supports de tests peuvent être des bandelettes de tests 501 (pour le reste de la description on parlera de bandelettes de tests). La cartouche 202 comprend un support rotatif 500, configuré pour être entrainé en rotation par la station 200. En utilisation normale de la cartouche 202 et du dispositif d'analyse d'urine 100, les bandelettes 501 demeurent montées dans le support rotatif 500 et ne se déplacent pas par rapport à ce dernier. Les bandelettes sont donc attachées et solidaires du support rotatif 500. En particulier, les bandelettes 501 ne sont pas enroulées de façon déroulable sur le support rotatif 500 pour l'utilisation : elles ne sont donc pas déroulées pendant l'utilisation.

Dans un mode de réalisation, le support rotatif 500 est de forme cylindrique creuse s'étendant annulairement autour d'un axe qui est, lorsque la cartouche 202 est montée dans la station 200, l'axe médian A du boitier 204 (par commodité de langage, on utilisera un seul axe A pour décrire les différents éléments, appelé l'axe de rotation A). En pratique le support rotatif est généralement symétrique de révolution autour de l'axe de rotation A. Le support rotatif 500 permet de stocker un nombre important de bandelettes de tests 501 tout en étant suffisamment compact pour être agencé à l'intérieur du boitier 204.

La cartouche 202 et le support rotatif 500 tels qu'illustrés sur les figures s'étendent sur un tour complet et peuvent effectuer dans la station 200 un tour complet. Toutefois, il peut être envisagé, pour des raisons d'encombrement ou pour permettre de libérer de l'espace pour d'autres composants, une cartouche 202 qui s'étend sur une portion de tour (par exemple moins de 180° ou 90°) et qui n'effectue en rotation qu'une portion de tour (par exemple moins de 270°). Dans ce cas, le nombre de bandelettes est typiquement moins élevé que pour le dispositif d'analyse d'urine illustré sur les figures.

Les bandelettes sont agencées selon un cercle ou une portion de cercle, par exemple à une extrémité radiale du support rotatif 500 pour maximiser leur nombre (plus le rayon est grand, plus le périmètre pour y installer des bandelettes est grand). Le positionnement en cercle assure que les bandelettes 501 sont toutes à la même distance de l'axe de rotation A et, de ce fait, de l'injecteur ou de l'analyseur (en particulier un capteur optique de l'analyseur, qui sera décrit plus tard). Cela assure aussi que le protocole de mesure pour chaque bandelette est identique. Comme illustré sur les figures, les bandelettes 501 peuvent être agencées généralement selon une disposition en cercle ou en arc de cercle. Ainsi les bandelettes 501 peuvent donc être à équidistance de l'axe de rotation A. Plus spécifiquement, chacune des bandelettes 501 peut être une petite bande peu épaisse et peu large qui s'étend dans son sens longitudinal parallèlement à l'axe A. Ainsi les bandelettes 501 sont disposées parallèlement entre elles.

Par sa forme et sa fonction, la cartouche 202 s'apparente à un barillet. Dans un exemple de réalisation, la cartouche occupe substantiellement le volume annulaire procuré par le logement annulaire 212. On prévoit un petit jeu fonctionnel pour permettre à la cartouche de tourner sans frotter contre les parois du logement annulaire 212.

En l'espèce, un diamètre extérieur du support rotatif 500 peut être compris entre 30 mm et 130 mm, de préférence environ 60 mm. Une hauteur du support rotatif, mesurée selon la direction de l'axe A peut être compris entre 12 mm et 40 mm, de préférence environ 14 mm. Un rapport entre le diamètre du support rotatif et le diamètre du boitier 204 peut être supérieur ou égal à 0,3, de préférence supérieur ou égal à 0,5. On obtient ainsi une solution de très bonne compacité au regard du nombre important de bandelettes de tests disponibles.

Le support rotatif 500 comprend une portion annulaire 502 et une portion cylindrique 504, s'étend depuis une extrémité radiale externe de la portion annulaire 502. La portion cylindrique 504 s'étend typiquement d'un seul côté de la portion annulaire 502 et est configurée pour s'insérer dans le logement annulaire 212 du boitier 204. Les bandelettes 501 sont positionnées le long de la portion cylindrique 504 (orientées parallèlement à l'axe de rotation A), afin de pouvoir défiler sélectivement et/ou successivement devant l'injecteur et l'analyseur. La portion annulaire 502, quant à elle, reste en dehors du logement annulaire 212 et permet notamment de rigidifier la portion cylindrique 504 et/ou de permettre l'entrainement en rotation de la cartouche 202.

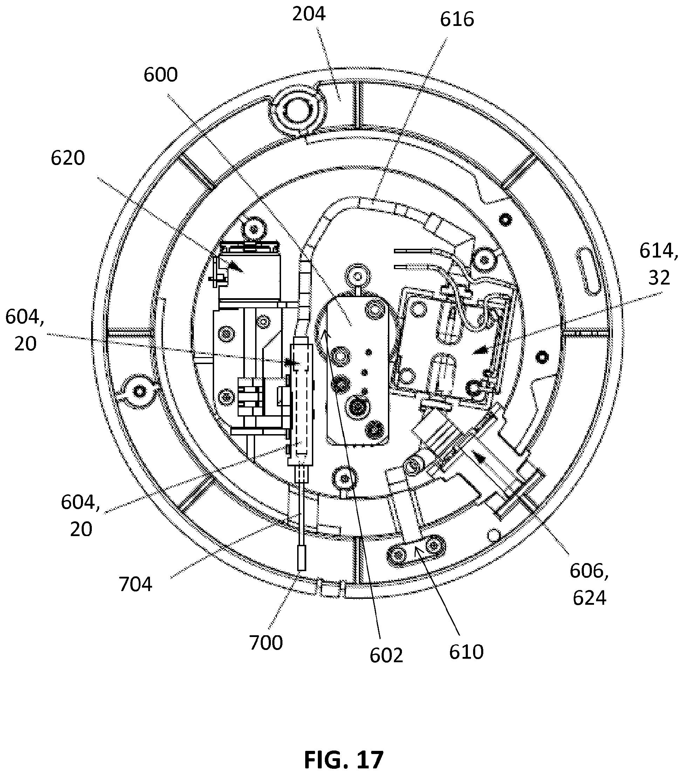

A cet effet, la portion annulaire 502 peut comprendre en outre un coupleur mécanique 506 configuré pour s'engager avec un coupleur mécanique de la station 200, par exemple un manchon femelle de fixation configuré pour s'engager avec un arbre entrainé en rotation par le moteur ou bien un arbre mâle de fixation configuré pour s'engager un manchon femelle entrainé en rotation par le moteur. Dans l'exemple illustré aux figures, il est prévu un pignon axial d'entrainement 602 (visible en figure 17) en sortie de réducteur, et la portion femelle est formée par le moyeu 506 du support rotatif 500.

Dans un mode de réalisation, le coupleur mécanique 506 est disposé sur l'axe de rotation A du support rotatif 500. Toutefois, dans l'exemple où la cartouche 202 est entrainée en rotation via un coupleur sur la portion cylindrique, la portion annulaire 502 peut être dépourvue du manchon femelle 602. La portion annulaire 502 pourrait être montée par tout type de liaison pivotante par rapport au boitier 204. La portion annulaire 502, à l'exception du coupleur mécanique 506, lorsqu'il est traversant, au niveau de l'axe A, peut s'apparenter à un disque. Le manchon femelle et le moyeu peuvent être inversés.

Dans un mode de réalisation, la cartouche 202 comprend un séparateur 508 comportant des logements 510 permettant de recevoir les bandelettes 501. Les logements 510 et les bandelettes 501 ont des dimensions similaires qui seront données en fin de description.

Le séparateur 508 est par exemple une pièce flexible, notamment en élastomère, en forme de bande ou ruban destinée à être enroulée dans le support rotatif 500. Le séparateur 508 s'étend le long d'une direction longitudinale et peut être enroulés contre une paroi interne de la portion annulaire 504 du support rotatif 500. Le séparateur 508 comprend une première face comprenant une pluralité de logements 510 recevant chacun un ou plusieurs bandelettes de test 501. La première face peut être recouverte par un opercule pour protéger les bandelettes hors utilisation. Le séparateur 508 comprend une deuxième face comprenant, en regard de chaque logement 510, au moins un orifice traversant 512 (par exemple deux, comme illustré sur la vue en transparence de la portion annulaire 504). Les logements 510 sont ainsi scellés. Grâce au séparateur 508 flexible, l'insertion des bandelettes de test 501 dans les logements 510 peut se faire sur une surface plane, ce qui simplifie le montage. Une fois le séparateur 508 installé, les logements 510 s'étendent parallèlement à la direction de rotation A.

La portion cylindrique 504 est transparente, ou bien comprend des zones transparentes, notamment en regard des logements 510. La portion cylindrique 504 est au contact du séparateur 508, en particulier la deuxième face du séparateur 508. Lors d'une analyse par colorimétrie, la lumière peut traverser la portion cylindrique 504, en passant par l'orifice traversant 512 pour analyser les bandelettes de test.

Le support rotatif 500, et plus particulièrement la portion cylindrique 504, comprend en outre une ouverture de purge 514, traversante, pour permettre à l'injecteur de traverser la cartouche 202 et le logement annulaire 212 et de rejoindre un circuit d'évacuation de la station. Le séparateur 508 ne recouvre pas l'ouverture de purge 514. Ce circuit sera décrit en détail plus tard.

La cartouche 202 comprend en outre un identificateur 516, représenté sur la figure 16 par une puce RFID. L'identificateur 516 permet à la station 200 de savoir quelle cartouche 202 a été insérée. L'identification 516 est typiquement un tag passif RFID.

Chaque logement 510 peut recevoir une unique bandelette de test. Toutes les bandelettes de test 501 des logements 510 peuvent être d'un même type. Par même type, on entend qu'elles sont sensibles aux mêmes composés contenus dans l'urine. La cartouche 202 est alors adapté pour une analyse spécifique. En variante, la bandelette de test 501 reçue dans le logement 510 peut être d'un type différent de la bandelette de test reçue dans le logement voisin. Ainsi, plusieurs types d'analyses, requérant différents types de bandelettes de test, peuvent être réalisées à partir de la même cartouche 202. En variante, chaque logement 510 peut contenir une pluralité de bandelettes de test 501 de types différents. Ainsi, plusieurs types d'analyses peuvent être réalisées à partir d'un même logement.

Comme indiqué précédemment, chaque logement 510 est recouvert et fermé par un opercule. L'opercule permet d'isoler hermétiquement les bandelettes de test 501 reçues dans un logement 510 de l'environnement extérieur et de logements voisins. Le logement 510 devient accessible pour l'injection de liquide typiquement par percement de l'opercule (par exemple par l'injecteur 604. Alors, avant une analyse, les réactifs des bandelettes de test sont protégés d'une éventuelle contamination. En outre, après une analyse, l'opercule peut contenir de l'urine introduite dans le logement 510. L'opercule est ici en un matériau inerte. Par exemple, l'opercule peut être en silicone ou en acrylique. De préférence, l'opercule est de qualité médicale, pour éviter la contamination des bandelettes de tests avec des produits indésirables contenus dans l'opercule. Alors, les réactifs des bandelettes de test sont conservés intacts avant une analyse. En outre, l'opercule est transparent, un taux de transparence étant de préférence supérieur à 99%. Alors, une analyse par colorimétrie peut être réalisée sur une bandelette de test au travers l'opercule.

### Actionneur d'entrainement

La cartouche 202 est, lorsque mise en place dans la station 200, couplée mécaniquement à un actionneur d'entrainement 600 (figure 17), via son coupleur mécanique 506 et un coupleur mécanique complémentaire 602 de la station 200, pour être entrainé en rotation autour de l'axe A. La cartouche 202 peut alors être sélectivement positionnée pour aligner une bandelette de test face à l'injecteur 604 ou l'analyseur 606. L'usage de la cartouche 202 permet d'avoir un équipage mobile simple avec un seul axe de rotation.

L'actionneur d'entrainement 600 peut être déporté par rapport à l'axe A et un train d'engrenages (pouvant aussi faire office de réducteur) permet d'entrainer le coupleur mécanique complémentaire 602. Le coupleur mécanique 602 complémentaire de la station 200 se trouve sur l'axe de rotation A.

L'actionneur d'entrainement 600 peut entrainer la cartouche 202 selon la direction horaire ou antihoraire. La cartouche peut alors rapidement atteindre la position désirée, suivant la trajectoire la plus courte. Ainsi, on réduit encore les contraintes liées au positionnement et l'agencement de l'injecteur 604 et de l'analyseur 606.

L'actionneur d'entrainement 600 est par exemple un moteur d'entrainement, comme un moteur pas-à-pas ou un moteur à courant continu. Alternativement, l'actionneur d'entrainement 600 peut impliquer un système hydraulique ou autre.

### Circuit fluidique

L'orifice de collecte 218 est fluidiquement connecté à un collecteur 610 puis un tuyau de collecte puis une pompe 614, puis un tuyau d'acheminement 616 puis l'injecteur 604. L'injecteur 604 est mobile dans le boitier 204, de sorte que l'injecteur peut se déplacer pour injecter de l'urine sélectivement sur une bandelette ou dans un tuyau de vidange 618 relié à l'orifice de vidange 310, en fonction de la position de la cartouche 202 dans la station 200. L'urine collectée par l'orifice de collecte 218 est mise en mouvement dans le circuit fluidique grâce à la pompe 614. Le tuyau d'acheminement 616 peut comprendre une portion souple, notamment à son extrémité qui le connecte à l'injecteur, pour accommoder le déplacement de l'injecteur.

La correspondance entre le circuit fluidique 30 de la figure 3 et la station 200 se fait comme suite : la pompe 614 et la pompe 32, le collecteur 610, le tuyau de collecte 612 et le réservoir 34 ; le tuyau d'acheminement 616 et les tuyau 36 (entre la pompe 32 et le capteur 10 ; l'injecteur 604 et l'injecteur 20.

L'injecteur 604 comprend typiquement trois positions : une position de retrait PR, une position d'injection PI et une position de purge PP. En position de retrait PR, l'injecteur 604 ne gêne pas la rotation de la cartouche 202 dans le logement annulaire 212 ; en position d'injection PI, l'injecteur 604 permet l'injection d'urine sur une bandelette positionnée dans la zone d'injection ZI ; en position de purge PP, l'injecteur 604 est relié au tuyau de vidange 618. Typiquement, en position de retrait PR, l'injecteur 604 est entièrement radialement interne au logement annulaire 212, entièrement en retrait ; en position d'injection PI, l'injecteur 604 est partiellement interne au logement annulaire 212 et partiellement dans le logement annulaire 212 ; en position de purge, l'injecteur 604 est partiellement interne au logement annulaire 212, partiellement dans le logement annulaire 212 et partiellement externe au logement annulaire 212. Pour que l'injecteur puisse être en position de purge PP, la cartouche 202 doit être elle aussi être en position de purge, c'est-à-dire que l'orifice de purge 514 du support rotatif 500 doit être aligné avec l'injecteur 604 (radialement aligné avec une extrémité distale d'injection de l'injecteur).

La pompe 614 peut aspirer de l'urine. La pompe aspire par exemple entre 5 microlitres et 1 mL, de préférence environ 20 microlitres. De plus, la pompe permet d'acheminer un volume suffisant d'urine vers l'injecteur 604 pour pouvoir réaliser une analyse concluante. Un débit d'aspiration de la pompe 614 est choisi en fonction du diamètre de l'orifice de collecte. Avantageusement, la pompe peut aspirer de l'urine depuis l'orifice de collecte vers l'injecteur sans former de bulles d'air.

La pompe 614 peut être de différents types possibles. La pompe 614 peut être une pompe péristaltique miniaturisée. La pompe 614 peut être un système de pompe pneumatique miniaturisée comme détaillé ci-après. Dans le cas où la pompe 614 est une pompe pneumatique miniaturisée, ce système pneumatique est configuré pour créer une dépression afin d'aspirer l'urine depuis l'orifice de collecte 218, puis une pression positive pour pousser l'urine vers l'injecteur 604 et le canal de purge. La pompe du système pneumatique peut être ici une pompe de type rotative, le sens de rotation fournissant respectivement et sélectivement une dépression ou une surpression. La pompe du système pneumatique peut être aussi une pompe de type piézoélectrique, comme décrite précédemment.

La solution présentée permet de contrôler de manière précise le volume acheminé dans le dispositif d'analyse d'urine, notamment grâce au capteur décrit plus haut (incorporé à l'injecteur 604).

L'injecteur 604 est typiquement configuré pour être connectée au tuyau d'acheminement 616. L'injecteur 604 peut comprend une aiguille 704 et l'extrémité distale 700 peut alors être l'extrémité de l'aiguille 704. Afin de pouvoir injecter de l'urine sur les bandelettes 501, l'injecteur 604 est mobile en translation selon une direction de translation par rapport au boitier 204. Un actionneur de déplacement 620 est prévu dans le boitier 204 pour faire déplacer l'injecteur. L'injecteur 604 peut être positionné, dans le boitier 204, radialement à l'intérieur du logement annulaire 212. De la même façon, l'actionneur de déplacement 620 peut être lui aussi positionné radialement à l'intérieur du logement annulaire 212.

Alternativement, l'actionneur de déplacement 620 peut être un moteur linéaire, voire un actionneur linéaire (vérin, etc.), qui produit un mouvement en translation directement.

L'actionneur de déplacement 620 permet de déplacer l'injecteur 604 dans les trois positions mentionnées précédemment : la position de retrait PR, la position d'injection PI, la position de purge PP. D'autres types de moteurs et/ou liaisons mécaniques permettent de déplacer l'injecteur 604 en translation.

L'extrémité distale 700 peut aussi être configurée pour percer l'opercule de la cartouche 202. Un biseau peut être prévu pour faciliter l'insertion. L'aiguille 704 peut avoir un diamètre d'environ 0,5mm. L'injecteur 604 peut injecter un volume contrôlé d'urine sur une bandelette de test, par exemple entre 2,5 microlitres et 3,5 microlitres. L'injecteur injecte un volume suffisant d'urine sur une bandelette de test pour réaliser une analyse concluante sans risquer un débordement d'urine du logement.

L'injecteur 604 peut ici être l'injecteur 20 tel que décrit auparavant. La mesure du volume de référence Vref et du débit de la pompe 614 permettent d'effectuer une injection précise et répétable, malgré le fait que l'urine collectée soit un fluide mixte.

### Capteur de position et injecteur

Un capteur de position 624 peut être prévu dans le boitier 204 pour positionner la cartouche par rapport à l'injecteur 604 et/ou l'analyseur 606. Cela permet d'améliorer la précision puisque l'actionneur de déplacement 620 peut être piloté par des consignes de position utilisant les données du capteur de position 624 (boucle de rétroaction).

L'analyseur 606 peut réaliser l'analyse par colorimétrie sur les supports de test. On entend par « analyse par colorimétrie » une mesure d'absorbance ou de fluorescence sous un éclairage prédéterminé, en transmission on en réflexion. L'analyseur 606 peut alors déterminer un ou plusieurs résultats d'analyses.

L'analyseur 606 peut être un analyseur optique, comprenant au moins une source de lumière (par exemple un ou plusieurs diodes électroluminescentes) et un capteur (par exemple une photodiode CCD, *« Charged Coupled Device »,* ou CMOS (« *Complementary Metal Oxide Semiconductor »* La source de lumière peut comprendre deux sources distinctes (par exemple deux longueurs d'onde différentes)

L'analyseur 606 est typiquement situé de part et d'autre du logement annulaire, de sorte que la lumière émise par la source de lumière puisse traverser la portion cylindrique transparente, puis les orifices du séparateur, puis la bandelette, pour enfin atteindre le capteur optique. Un séparateur optique permet de guider les deux sources de lumière pour éviter les fuites de lumière d'un chemin optique à l'autre.

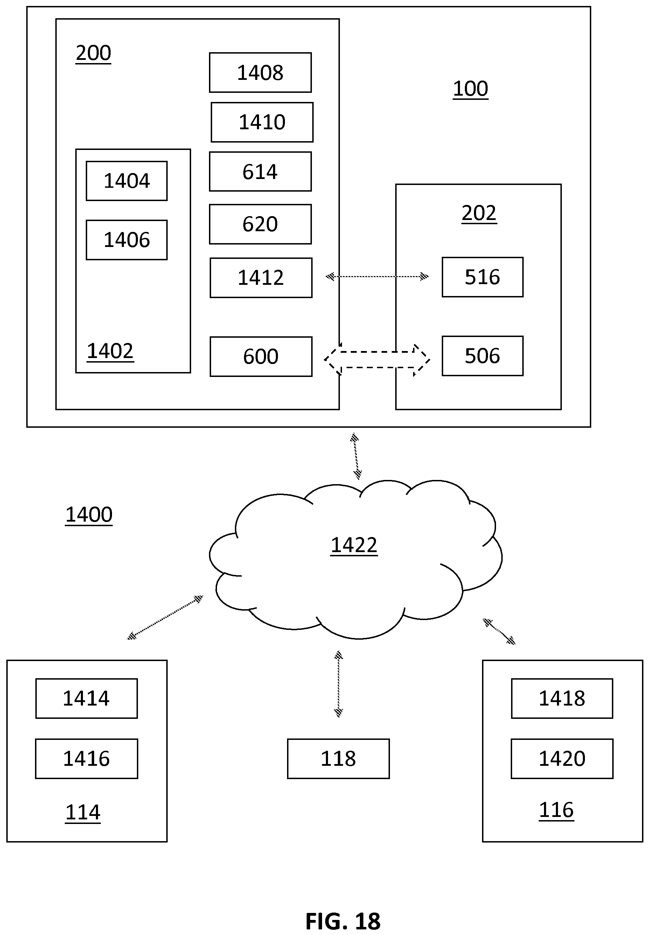

La figure 18 représente une vue schématique d'un environnement d'analyse 1400 comprenant le dispositif d'analyse d'urine et de son environnement. La station 200 est contrôlée par une unité électronique de contrôle ECU 1402. L'ECU 1402 est à l'intérieur du boitier 204. L'ECU 1402 permet de contrôler les composants de l'ensemble de test pour réaliser une analyse d'urine exploitant les bandelettes de test 501 et obtenir un ou plusieurs résultats d'analyses. En particulier, l'ECU 1402 gère l'actionneur d'entrainement 600, l'actionneur de déplacement 620, le capteur de position 624, l'analyseur 606, la pompe 32/614 et le capteur 20.

L'ECU 1402 comprend typiquement un processeur 1404 et une mémoire 1406 apte à stocker des instructions que le processeur 1404 exécute. En particulier, les procédés décrits dans la description sont stockés sous forme de lignes d'instructions dans la mémoire 1406. Afin de pouvoir communiquer directement ou indirectement avec le terminal mobile 114, le serveur 116 et/ou, le cas échéant, l'activateur externe 118, la station 200 comprend un module de communication 1408, typiquement sans-fil, par exemple Bluetooth, WiFi et/ou cellulaire (GSM, 3G, 4G, 5G,4G-LTE). Une batterie 1410 permet d'alimenter en énergie les différents composants de la station 200. Pour identifier la cartouche 202 dans la station 200 (via l'identification 516), la station 200 peut comprendre un lecteur 1412, par exemple un lecteur de proximité sans contact, comme un lecteur RFID. L'ECU 18 du circuit fluidique 30 est typiquement comprise dans l'ECU 1402, qui fait alors office d'ECU principale.

Le terminal mobile 114 comprend notamment un processeur 1414 et un mémoire 1416, qui permettent par exemple de faire tourner une application qui sert d'interface utilisateur pour le dispositif 100. Le serveur 116 comprend également un processeur 1418 et une mémoire 1420, pour traiter et stocker des données générées notamment par le dispositif 100.

Le dispositif 100, le terminal mobile 114 et le serveur 116 communique entre eux par un réseau de télécommunication 1422. Le réseau de télécommunication 1422 peut être hybride, en ce qui comprend un réseau WiFi ou Bluetooh et un réseau cellulaire, dont les rôles ont été expliquées précédemment.

### Procédé de pilotage

En relation avec le dispositif d'analyse d'urine 100 présentés précédemment, différents procédés vont à présent être décrit. Les étapes décrites peuvent être toutes mises en oeuvre lors d'une analyse ou seulement certaines d'entre elles peuvent être mises en oeuvre. En particulier, ces étapes sont exécutables à partir d'instructions stockés dans la mémoire 1305 de l'ECU 1402.

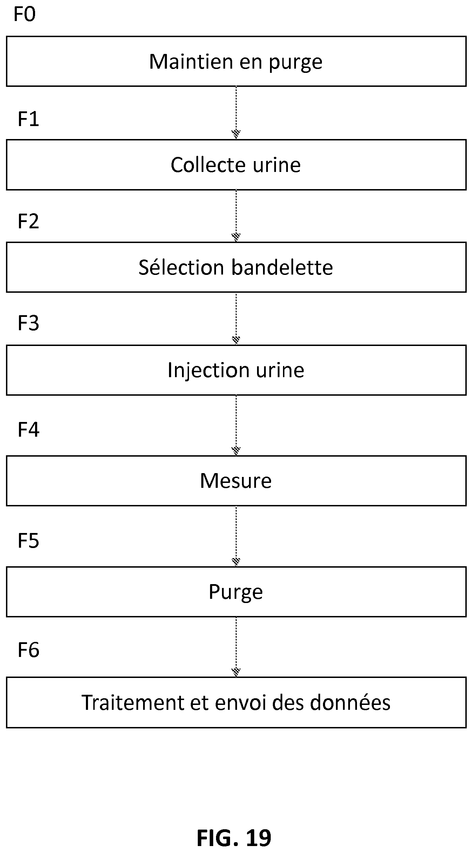

Un procédé global d'utilisation du dispositif 100 va être décrit en relation avec la figure 19.

L'étape F0 consiste à maintenir le dispositif d'analyse d'urine 100 dans une position de purge. L'ECU contrôle l'actionneur d'entrainement 600 pour mettre l'ouverture de purge 514 de la cartouche 202 dans la zone d'injection ZI (i.e. alignée avec l'injecteur 604). A cette fin, l'ECU utilise des données de position obtenues par le capteur de position 624 qui peut repérer l'ouverture 512 (par exemple par un signal lumineux plus fort car il n'y a pas de bandelette entre la source lumineuse et la photodiode). Avant la rotation de la cartouche, l'ECU a piloté l'actionneur de déplacement 620 pour mettre l'injecteur en position de retrait PR. Une fois l'ouverture de purge 514 au regard de l'injecteur, l'ECU pilote l'actionneur de déplacement 620 pour que l'injecteur 604 passe en position de purge PP. Le dispositif 100 peut ainsi être rincé de l'eau ou de résidus d'urine. Ainsi, de l'urine ou de l'eau reçu des toilettes dans le tuyau de collecte 612 peut rejoindre l'orifice de vidange 310.

Dans une étape F1 (« collecte urine »), l'ECU active la pompe 614 pour acheminer de l'urine depuis l'orifice de collecte 218 vers l'injecteur 604. L'injecteur 604 est toujours en position de purge PP et de l'urine peut ressortir par l'orifice de vidange. Cette étape permet de nettoyer et de s'assurer que l'injecteur est bien empli d'urine. Durant cette étape F1, les étapes E0 à E4 sont effectuées. L'étape F1 peut aussi comprendre une étape de pré-charge (telle que décrite précédemment), pour amener une quantité contrôlée d'urine au niveau de l'extrémité distale 700 de l'injecteur 604.

L'étape F2 (« sélection bandelette ») consiste à positionner le dispositif d'analyse d'urine 100 dans une position de sélection, en mettant la bandelette 501 désirée en regard de l'injecteur 604. L'ECU active l'actionneur de déplacement 620 pour déplacer l'injecteur 604 de la position de purge PP vers la position de retrait PR. L'ECU pilote ensuite l'actionneur d'entrainement 600 pour que la cartouche 202 soit mise en rotation et que la bandelette 501 choisie soit mise dans la zone d'injection ZI. Le choix de la bandelette 501 peut dépendre de l'analyse désirée (si différents types de bandelettes sont mis dans la cartouche 202) par l'utilisateur ou peut être automatiquement fait (bandelettes utilisées successivement selon un programme pré-établi). L'ECU récupère à nouveau les données du capteur de position 624 pour assurer que la bandelette désirée soit correctement placée. L'étape F2 peut prendre quelques secondes. Néanmoins, comme l'urine a déjà été collectée à l'étape F1, il n'y a pas de risque de manquer la miction de l'utilisateur.

L'étape F3 (« injection urine ») consiste par la suite à positionner le dispositif d'analyse d'urine dans une position d'injection et à injecter de l'urine. L'ECU peut piloter l'actionneur d'entrainement 600 puis pilote l'actionneur de déplacement 620 pour que l'injecteur passe en position d'injection PI. L'extrémité distale 700 perce alors l'opercule de la cartouche 202. L'ECU pilote ensuite la pompe 614 pour injecter de l'urine sur une bandelette de test. L'urine injectée peut alors réagir avec les réactifs de la bandelette de test. Après l'injection effective, l'injecteur 604 peut être rétracté en position de retrait PR. L'étape F3 comprend donc l'étape E5.

L'étape F4 (« mesure ») est une étape de mesure qui consiste à obtenir des données sur la bandelette désirée avec l'analyseur 606. A cette fin, l'ECU pilote l'analyseur 606 pour qu'il génère des données sur les bandelettes.

L'étape F5 (« purge ») consiste à purger le dispositif d'analyse d'urine. L'ECU pilote l'actionneur d'entrainement 600 pour aligner l'orifice de purge 514 avec l'injecteur 604, puis pilote l'actionneur de déplacement 620 pour mettre l'injecteur en position de purge PP, puis active la pompe 614 pour pousser de l'air. Alors, l'urine est expulsée du dispositif d'analyse d'urine via le canal de purge et par l'orifice de vidange 310. Le dispositif d'analyse d'urine 100 est ainsi à la même position qu'en étape F0. Au préalable de l'alignement de l'orifice de purge 514 avec l'injecteur 604, l'injecteur 604 peut être remis en position de retrait PR en pilotant l'actionneur de déplacement 620, si cela n'a pas été fait à la fin de l'étape E3.

L'analyseur réalise une analyse par colorimétrie sur la bandelette de test. Le dispositif d'analyse d'urine en déduit le ou les résultats d'analyses. L'analyse réalisée dépend du type de bandelette de test. L'analyse réalisée peut également dépendre d'un choix de l'utilisateur. L'analyse effectuée peut également être choisie en fonction de l'utilisateur identifié.

Une étape F6 de traitement des données et de transmission du ou des résultats peut être mise en oeuvre. L'ECU traite les données reçues par l'analyseur 606 et instruit un émetteur de transmettre le ou les résultats, par exemple directement au terminal mobile 114 de l'utilisateur. Le ou les résultats peuvent également être envoyés vers le serveur 116. L'utilisateur peut par exemple visualiser et exploiter le ou les résultats sur l'application du smartphone 114, ou sur un site web. Le ou les résultats peuvent également être envoyés vers un professionnel de santé. L'étape F6 peut être effectué à n'importe quel moment après l'étape F4.

L'étape F0 consiste à maintenir le dispositif d'analyse d'urine dans la position de purge, en mettant l'injecteur 604 en position de purge PP. Complément sur les supports de test

Les supports de test comprennent un réactif qui réagit au contact de l'urine. Dans un mode de réalisation, le réactif est un réactif sec. En particulier, les supports de test sont des bandelettes de test, qui vont être décrites plus en détail ci-dessous.

Les bandelettes de test 501 peuvent être du type immunodosage à flux latéral ou vertical, en anglais « *lateral or vertical flow immunoassay ».* Alors, les bandelettes de test 501 comportent un tampon d'échantillonnage et un tampon d'absorption. Une membrane de nitrocellulose s'étend entre le tampon d'échantillonnage et le tampon d'absorption. Alors, lorsqu'un échantillon d'urine est introduit sur le tampon d'échantillonnage, il migre par capillarité jusqu'au tampon d'absorption en passant au travers d'un tampon conjugué, d'une ou plusieurs lignes de test, et d'une ligne de contrôle. Le tampon conjugué, le ou les lignes de test et la ligne de contrôle contiennent des réactifs.

Le tampon conjugué comporte notamment des anticorps de détection sensibles à des composés contenus dans l'urine. Si les composés sont présents lorsque l'échantillon d'urine traverse le tampon conjugué, alors les anticorps se fixent aux composés pour former des marqueurs. Les marqueurs migrent vers une ligne de test. La ligne de test comporte notamment des anticorps de test. Les anticorps de test se lient avec les marqueurs et les retiennent sur la ligne de test. Alors, une ligne colorée se forme et la densité de la ligne varie en fonction de la concentration de marqueurs présents. L'échantillon restant migre vers une ligne de contrôle. La ligne de contrôle comporte des anticorps de contrôle, permettant d'indiquer que l'échantillon a traversé la membrane de nitrocellulose.

Par exemple, les bandelettes de test peuvent être des bandelettes de type ELISA. Ce type de bandelette de test permet une détection de l'hormone de grossesse hCG dans l'urine. Alors, l'anticorps de détection peut être « mouse monoclonal beta hCG », l'anticorps de test peut être « goat poluclonal anti-mouse IgG » et l'anticorps de contrôle peut être « rabbit polyclonal anti-mouse igG ».

Les bandelettes de test peuvent être de type bandelettes colorimétriques classiques. Alors, chaque bandelette de test comporte au moins un tampon contenant un ou plusieurs réactifs sensibles à un ou plusieurs composés contenus dans l'échantillon d'urine. Par exemple, le ou les composés peuvent être : l'hormone LH, l'hormone HCG, les leucocytes/nitrites, les urobilinogène/bilirubine, les protéines, le pH, la gravité spécifique et/ou le glucose.

D'autres types de réactions peuvent utiliser réactifs ou composés conçus pour détecter la présence d'un analyte particulier (par exemple des polymères à empreinte moléculaire "MIP" ou "Molecularly imprinted polymers"), notamment un principe actif de médicament ou un métabolite de principe actif de médicament dans l'urine. Dans ce cas, le dispositif peut être utilisé pour contrôler l'observance d'un utilisateur pour un traitement médicamenteux, notamment vérifier qu'il prend bien son traitement ou l'alerter lorsqu'il a omis de le prendre.

Chaque bandelette de tests est globalement rectangulaire. Une largeur de chaque bandelette de test peut être comprise entre 0,5 mm et 3 mm, par exemple environ 1 mm. Une longueur de chaque bandelette de test peut être comprise entre 10 et 15 mm, par exemple 12 mm ou environ 12 mm. Alternativement, chaque bandelette de test peut avoir une forme quelconque, par exemple carrée ou circulaire. La forme et les dimensions des bandelettes de test permettent de stocker un nombre important de bandelettes de test dans le dispositif d'analyse d'urine (au moins 50 bandelettes, voire au moins 100 bandelettes). En effet, il apparait même possible de stocker jusqu'à 120 bandelettes de test, ce qui correspond à 4 mois d'analyses lorsqu'un utilisateur réalise une analyse par jour.

## Revendications

1. Capteur de volume et de débit (10) comprenant :
- un conduit (12) apte à recevoir un fluide mixte,
- une première sonde (14) apte à identifier, localement dans le conduit, un changement de phase du fluide mixte au niveau d'une première position (P1) dans le conduit (12), la première position (P1) étant utilisée pour définir un volume de référence (Vref) du conduit,
- une deuxième sonde (16) apte à identifier, localement dans le conduit, un changement de phase du fluide mixte au niveau d'une deuxième position (P2) dans le conduit (12), la deuxième position (P2) étant espacée de la première position (P1) le long du conduit (12), la première position (P1) et la deuxième position (P2) définissant entre elles le volume de référence (Vref) du conduit,
- une unité de contrôle électronique ECU (18) comprenant des instructions pour :
. déterminer, en identifiant les changements de phase par la première sonde (14) et la deuxième sonde, que le volume de référence (Vref) est empli de liquide, et,
. déterminer une valeur (N) d'une grandeur physique de déplacement (Nb, T) liée au déplacement du liquide entre la première position (P1) et la deuxième position (P2).

2. Capteur de volume et de débit (10) selon la revendication 1, dans lequel la première position (P1) et la deuxième position (P2) définissent un volume de contrôle (Vc) du conduit (12), le volume de contrôle (Vc) étant le même que le volume de référence (Vref), l'ECU comprenant des instructions pour :
. déterminer un débit en utilisant la valeur déterminée (N) de la grandeur physique de déplacement déterminée (Nb, T) et la valeur du volume de contrôle (Vc).

3. Capteur de volume et de débit (10) selon la revendication 1 ou 2, dans lequel l'ECU comprend des instructions pour effectuer les étapes suivantes :
. (E1) détection d'un changement de phase vers une phase liquide, par la première sonde (14),
. (E2) détection d'un passage d'un changement de phase vers une phase liquide par la deuxième sonde (16),
. (E3) détermination, à l'aide d'un compteur, de la valeur de la grandeur physique de déplacement (N), qui correspond à la grandeur physique écoulée entre la détection du changement de phase par la première sonde (14) et la détection du changement de phase par la deuxième sonde (16).

4. Capteur de volume et de débit (10) selon la revendication 3, dans lequel l'étape de détermination de la valeur (N) de la grandeur physique de déplacement comprend (E3) :
- en réponse à l'étape de détection (E1) par la première sonde, (E31) démarrage d'un compteur, ledit compteur étant associé au changement de phase détecté,
- en réponse à l'étape de détection (E2) par la deuxième sonde, (E32) arrêt du chronomètre associé au changement de phase détecté.

5. Capteur de volume et de débit (10) selon la revendication 2, dans lequel l'ECU (18) stocke une valeur du volume de contrôle (Vc) et des instructions pour effectuer les étapes suivantes :
- (E4) calcul d'un débit en utilisant la valeur déterminée (N) de la grandeur physique de déplacement déterminée (Nb, T) et la valeur du volume de contrôle (Vc),
- (E5) génération d'une consigne d'injection pour une pompe en utilisant le débit déterminé et une consigne prédéterminée, pour injecter une quantité souhaitée de liquide.

6. Capteur de volume et de débit (10) selon l'une quelconque des revendications 1 à **4,** dans lequel la grandeur physique de déplacement est :
- un temps ou
- un nombre de coups de pompe lorsque la revendication 6 dépend des revendications 1 à 4 et un nombre de coups de pompe de la pompe lorsque la revendication 6 dépend de la revendication 5, la pompe mettant le fluide en mouvement dans le conduit.

7. Capteur de volume et de débit (10) selon l'une quelconque des revendications 1 à 6, dans lequel la détermination que le volume de référence est empli de liquide et la détermination de la valeur de la grandeur physique de déplacement sont effectuées en même temps.

8. Capteur de volume et de débit selon la revendication 7 en combinaison avec la revendication 3 **ou 4,** dans lequel l'ECU comprend des instructions pour effectuer les étapes suivantes :
. (E2a) détermination que la première sonde (14) n'a pas détecté d'autre changement de phase entre, temporellement, les deux détections (E1, E2) de changement de phase par la première sonde (14) et la deuxième sonde (16)
. (E2b) en réponse à la détermination de l'étape (E2a), détermination que le volume de référence Vref est empli de liquide.

9. Capteur de volume et de débit (10) selon l'une quelconque des revendications 1 à 6, dans lequel la détermination que le volume de référence est empli de liquide est effectuée avant détermination de la valeur de la grandeur physique de déplacement.

10. Capteur de volume et de débit (10) selon l'une quelconque des revendications 1 à 9, dans lequel chaque sonde est choisie au moins parmi : un capteur optique, des électrodes couplées, un capteur capacitif, un capteur thermique.

11. Capteur de volume et débit (10) selon l'une quelconque des revendications 1 à 10, dans lequel la première sonde comprend une sonde optique et la deuxième sonde comprend une sonde optique.

12. Injecteur (20) comprenant :
- un capteur de volume et de débit (10) selon l'une quelconque des revendications 1 à 11,
- une buse d'injection (22) avec une extrémité distale d'injection (24), la buse d'injection étant connectée à une extrémité du conduit (12),
dans lequel la valeur du volume du conduit (12) entre la deuxième position (P2) et l'extrémité distale d'injection (24), appelé volume mort (Vm), est inférieure à la valeur du volume de référence (Vref) ou moins de deux fois la valeur du volume de référence.

13. Injecteur selon la revendication 12, dans lequel l'ECU stocke la valeur du volume mort (Vm) et les instructions comprennent en outre l'étape suivante :
- la consigne d'injection utilise le débit déterminé, une consigne prédéterminée et le volume mort (Vm).

14. Injecteur selon la revendication 12 ou 13, dans lequel l'ECU génère une consigne de précharge pour une pompe, d'un volume égal au volume mort Vm ou au (1+A%)Vm, A étant compris entre 0 et 50.

15. Circuit fluidique comprenant une pompe (32) et un capteur (10) selon l'une quelconque des revendications 1 à 11 ou un injecteur (20) selon l'une quelconque des revendications 12 à 14, la pompe (32) étant pilotée par l'ECU pour recevoir des consignes de précharge ou d'injection.

## Patentansprüche

1. Volumen- und Durchflusssensor (10), umfassend:
- eine Leitung (12), die dazu ausgelegt ist, ein gemischtes Fluid aufzunehmen,
- eine erste Sonde (14), die in der Lage ist, lokal in der Leitung an einer ersten Position (P1) in der Leitung (12) einen Phasenwechsel des gemischten Fluids zu erkennen, wobei die erste Position (P1) zur Definition eines Referenzvolumens (Vref) der Leitung verwendet wird,
- eine zweite Sonde (16), die in der Lage ist, lokal in der Leitung an einer zweiten Position (P2) in der Leitung (12) einen Phasenwechsel des gemischten Fluids zu erkennen, wobei die zweite Position (P2) entlang der Leitung (12) von der ersten Position (P1) beabstandet ist, wobei die erste Position (P1) und die zweite Position (P2) zwischen sich das Referenzvolumen (Vref) des Kanals definieren,
- eine elektronische Steuereinheit ECU (18), die Anweisungen umfasst für:
. durch Erkennen der Phasenwechsel mittels der ersten Sonde (14) und der zweiten Sonde festzustellen, dass das Referenzvolumen (Vref) mit Flüssigkeit gefüllt ist, und,
. Ermitteln eines Wertes (N) einer physikalischen Verdrängungsgröße (Nb, T), die mit der Verdrängung der Flüssigkeit zwischen der ersten Position (P1) und der zweiten Position (P2) zusammenhängt.

2. Volumen- und Durchflusssensor (10) nach Anspruch 1, wobei die erste Position (P1) und die zweite Position (P2) ein Kontrollvolumen (Vc) der Leitung (12) definieren, wobei das Kontrollvolumen (Vc) mit dem Referenzvolumen (Vref) übereinstimmt, und wobei die ECU Anweisungen umfasst, um:
. Ermittlung eines Durchflusses unter Verwendung des ermittelten Werts (N) der ermittelten physikalischen Verdrängungsgröße (Nb, T) und des Werts des Kontrollvolumens (Vc).

3. Volumen- und Durchflusssensor (10) nach Anspruch 1 oder 2, wobei die ECU Befehle zur Durchführung der folgenden Schritte umfasst:
. (E1) Erfassung eines Phasenübergangs in eine flüssige Phase durch die erste Sonde (14),
. (E2) Erfassen eines Übergangs von einer Phasenänderung in eine flüssige Phase durch die zweite Sonde (16),
. (E3) Bestimmung des Wertes der physikalischen Größe "Verschiebung" (N) mithilfe eines Zählers, der der physikalischen Größe entspricht, die zwischen der Erfassung des Phasenübergangs durch die erste Sonde (14) und der Erfassung des Phasenübergangs durch die zweite Sonde (16) verstrichen ist.

4. Volumen- und Durchflusssensor (10) nach Anspruch 3, wobei der Schritt der Bestimmung des Wertes (N) der physikalischen Größe "Verschiebung" (E3) umfasst:
- als Reaktion auf den Erfassungsschritt (E1) durch die erste Sonde (E31) das Starten eines Zählers, wobei der Zähler der erfassten Phasenänderung zugeordnet ist,
- als Reaktion auf den Erfassungsschritt (E2) durch die zweite Sonde (E32) das Anhalten der mit der erfassten Phasenänderung verbundenen Zeitmessung.

5. Volumen- und Durchflusssensor (10) nach Anspruch 2, wobei die ECU (18) einen Wert des Kontrollvolumens (Vc) sowie Anweisungen zur Durchführung der folgenden Schritte speichert:
- (E4) Berechnung eines Durchflusses unter Verwendung des ermittelten Werts (N) der ermittelten physikalischen Verdrängungsgröße (Nb, T) und des Werts des Kontrollvolumens (Vc),
- (E5) Erzeugung eines Einspritzsollwerts für eine Pumpe unter Verwendung des ermittelten Durchflusses und eines vorgegebenen Sollwerts, um eine gewünschte Flüssigkeitsmenge einzuspritzen.

6. Volumen- und Durchflusssensor (10) nach einem der Ansprüche 1 bis 4, wobei die physikalische Verdrängungsgröße:
- eine Zeit oder
- eine Anzahl von Pumpenhüben, wenn Anspruch 6 auf den Ansprüchen 1 bis 4 beruht, und eine Anzahl von Pumpenhüben der Pumpe, wenn Anspruch 6 auf Anspruch 5 beruht, wobei die Pumpe das Fluid in der Leitung in Bewegung versetzt.

7. Volumen- und Durchflusssensor (10) nach einem der Ansprüche 1 bis 6, wobei die Feststellung, dass das Referenzvolumen mit Flüssigkeit gefüllt ist, und die Ermittlung des Wertes der physikalischen Verdrängungsgröße gleichzeitig erfolgen.

8. Volumen- und Durchflusssensor nach Anspruch 7 in Kombination mit Anspruch 3 oder 4, wobei die ECU Befehle zur Durchführung der folgenden Schritte umfasst:
. (E2a) Feststellen, dass die erste Sonde (14) zeitlich zwischen den beiden Erfassungen (E1, E2) einer Phasenänderung durch die erste Sonde (14) und die zweite Sonde (16) keine weitere Phasenänderung erfasst hat
. (E2b) als Reaktion auf die Feststellung aus Schritt (E2a) Feststellung, dass das Referenzvolumen Vref mit Flüssigkeit gefüllt ist.

9. Volumen- und Durchflusssensor (10) nach einem der Ansprüche 1 bis 6, wobei die Feststellung, dass das Referenzvolumen mit Flüssigkeit gefüllt ist, vor der Bestimmung des Wertes der physikalischen Größe des Weges erfolgt.

10. Volumen- und Durchflusssensor (10) nach einem der Ansprüche 1 bis 9, wobei jede Sonde zumindest aus folgenden ausgewählt ist: einem optischen Sensor, gekoppelten Elektroden, einem kapazitiven Sensor, einem thermischen Sensor.

11. Volumen- und Durchflusssensor (10) nach einem der Ansprüche 1 bis 10, wobei die erste Sonde eine optische Sonde umfasst und die zweite Sonde eine optische Sonde umfasst.

12. Einspritzventil (20) mit:
- einen Volumen- und Durchflusssensor (10) gemäß einem der Ansprüche 1 bis 11,
- eine Einspritzdüse (22) mit einem distalen Einspritzende (24), wobei die Einspritzdüse mit einem Ende der Leitung (12) verbunden ist,
wobei der Wert des Volumens der Leitung (12) zwischen der zweiten Position (P2) und dem distalen Einspritzende (24), das als Totvolumen (Vm) bezeichnet wird, kleiner ist als der Wert des Referenzvolumens (Vref) oder weniger als das Doppelte des Wertes des Referenzvolumens beträgt.

13. Einspritzventil nach Anspruch 12, wobei die ECU den Wert des Totvolumens (Vm) speichert und die Anweisungen ferner den folgenden Schritt umfassen:
- der Einspritzsollwert verwendet den ermittelten Durchfluss, einen vorgegebenen Sollwert und das Totvolumen (Vm).

14. Einspritzventil nach Anspruch 12 oder 13, wobei die ECU einen Vorfüll-Sollwert für eine Pumpe erzeugt, dessen Volumen dem Totvolumen Vm oder (1+A%)Vm entspricht, wobei A zwischen 0 und 50 liegt.

15. Fluidkreislauf, umfassend eine Pumpe (32) und einen Sensor (10) gemäß einem der Ansprüche 1 bis 11 oder eine Einspritzdüse (20) gemäß einem der Ansprüche 12 bis 14, wobei die Pumpe (32) von der ECU angesteuert wird, um Vorladungs- oder Einspritzsollwerte zu empfangen.

## Claims

1. A volume and flow sensor (10) comprising:
- a conduit (12) adapted to receive a mixed fluid,
- a first probe (14) capable of detecting, locally within the conduit, a phase change in the mixed fluid at a first position (P1) in the conduit (12), the first position (P1) being used to define a reference volume (Vref) of the conduit,
- a second probe (16) capable of detecting, locally within the conduit, a phase change in the mixed fluid at a second position (P2) in the conduit (12), the second position (P2) being spaced apart from the first position (P1) along the conduit (12), the first position (P1) and the second position (P2) defining between them the reference volume (Vref) of the duct,
- an electronic control unit (ECU) (18) comprising instructions for:
. determining, by identifying the phase changes via the first probe (14) and the second probe, that the reference volume (Vref) is filled with liquid, and,
. determining a value (N) of a physical displacement quantity (Nb, T) related to the displacement of the liquid between the first position (P1) and the second position (P2).

2. The volume and flow sensor (10) according to claim 1, wherein the first position (P1) and the second position (P2) define a control volume (Vc) of the conduit (12), the control volume (Vc) being the same as the reference volume (Vref), the ECU comprising instructions for:
. determining a flow rate using the determined value (N) of the determined physical displacement quantity (Nb, T) and the value of the control volume (Vc).

3. The volume and flow sensor (10) according to claim 1 or 2, wherein the ECU includes instructions for performing the following steps:
. (E1) detecting a phase change to a liquid phase by the first probe (14),
. (E2) detecting the completion of a phase change to a liquid phase by the second probe (16),
. (E3) determining, using a counter, the value of the physical quantity of displacement (N), which corresponds to the physical quantity elapsed between the detection of the phase change by the first probe (14) and the detection of the phase change by the second probe (16).

4. The volume and flow rate sensor (10) according to claim 3, wherein the step of determining the value (N) of the physical quantity of displacement comprises (E3):
- in response to the detection step (E1) by the first probe, (E31) starting a counter, said counter being associated with the detected phase change,
- in response to the detection step (E2) by the second probe, (E32) stopping the timer associated with the detected phase change.

5. The volume and flow rate sensor (10) according to claim 2, wherein the ECU (18) stores a value for the control volume (Vc) and instructions for performing the following steps:
- (E4) calculating a flow rate using the determined value (N) of the determined physical displacement quantity (Nb, T) and the control volume value (Vc),
- (E5) generating an injection setpoint for a pump using the determined flow rate and a predetermined setpoint, to inject a desired quantity of liquid.

6. The volume and flow rate sensor (10) according to any one of claims 1 through 4, wherein the physical displacement quantity is:
- a time or
- a number of pump strokes when claim 6 depends on claims 1 through 4, and a number of pump strokes of the pump when claim 6 depends on claim 5, the pump causing the fluid to move through the conduit.

7. The volume and flow rate sensor (10) according to any one of claims 1 through 6, wherein the determination that the reference volume is filled with liquid and the determination of the value of the physical displacement quantity are performed simultaneously.

8. The volume and flow sensor according to claim 7 in combination with claim 3 or 4, wherein the ECU includes instructions for performing the following steps:
. (E2a) determining that the first sensor (14) has not detected any other phase change between, in time, the two phase change detections (E1, E2) by the first sensor (14) and the second sensor (16)
. (E2b) in response to the determination in step (E2a), determining that the reference volume Vref is filled with liquid.

9. The volume and flow rate sensor (10) according to any one of claims 1 through 6, wherein the determination that the reference volume is filled with liquid is made prior to determining the value of the physical quantity of displacement.

10. The volume and flow sensor (10) according to any one of claims 1 through 9, wherein each probe is selected from at least one of: an optical sensor, coupled electrodes, a capacitive sensor, and a thermal sensor.

11. The volume and flow sensor (10) according to any one of claims 1 through 10, wherein the first probe comprises an optical probe and the second probe comprises an optical probe.

12. An injector (20) comprising:
- a volume and flow sensor (10) according to any one of claims 1 through 11,
- an injection nozzle (22) with a distal injection end (24), the injection nozzle being connected to one end of the conduit (12),
wherein the volume of the conduit (12) between the second position (P2) and the distal injection end (24), referred to as the dead volume (Vm), is less than the reference volume (Vref) or less than twice the reference volume.

13. The injector according to claim 12, wherein the ECU stores the value of the dead volume (Vm) and the instructions further comprise the following step:
- the injection setpoint uses the determined flow rate, a predetermined setpoint, and the dead volume (Vm).

14. The injector according to claim 12 or 13, wherein the ECU generates a precharge setpoint for a pump, with a volume equal to the dead volume Vm or to (1+A%)Vm, where A is between 0 and 50.

15. A fluidic circuit comprising a pump (32) and a sensor (10) according to any one of claims 1 through 11 or an injector (20) according to any one of claims 12 through 14, wherein the pump (32) is controlled by the ECU to receive precharge or injection setpoints.
